(19) Europäisches Patentamt
European Patent Office
Office européen des brevets

(11) Veröffentlichungsnummer: 0 146 810 A2

# (12) EUROPÄISCHE PATENTANMELDUNG

(21) Anmeldenummer: 84114415.7

(22) Anmeldetag: 28.11.84

(51) Int. Cl.⁴: C 07 H 15/04
A 61 K 31/70

(30) Priorität: 05.12.83 CH 6493/83
28.09.84 CH 4671/84

(43) Veröffentlichungstag der Anmeldung: 03.07.85 Patentblatt 85/27

(84) Benannte Vertragsstaaten: AT BE CH DE FR GB IT LI LU NL SE

(71) Anmelder: Solco Basel AG
Gellertstrasse 18
CH-4052 Basel(CH)

(72) Erfinder: Tschannen, Roland, Dr.
Gerbergässlein 27
CH-4051 Basel(DE)

(72) Erfinder: Fraefel, Wolfgang, Prof. Dr.
Au Noyeret CH-1772
Grolley(DE)

(72) Erfinder: Schmidt, Richard R., Prof. Dr.
Grossherzog-Friedrich-Strasse 11
D-7750 Konstanz 16(DE)

(72) Erfinder: Kläger, Rudolf
Haupstrasse 67
D-7241 Eutingen i. G.(DE)

(72) Erfinder: Zimmermann, Peter
Ruhesteinweg 14
D-7730 Villingen(DE)

(74) Vertreter: Frossard, Michel, Dr. et al,
A. Braun, Braun, Héritier, Eschmann AG, Patentanwälte
Holbeinstrasse 36-38
CH-4051 Basel(CH)

## (54) Sphingosinderivate, ihre Herstellung und pharmazeutische Zubereitung.

(57) Beschrieben werden neue Verbindungen der Formel (I)-D und (I)-L gemäss Formelblatt, beispielsweise D- bzw. L-erythro-1-(β-D-Glucopyranosyloxy)-3-hydroxy-2-palmitoylamino-4-trans-octadecen, welche eine wundheilungsfördernde bzw. zell- und geweberegenerierende Wirkung ausüben und zur Behandlung von Wunden jeglicher Genese therapeutisch verwendet werden können. Sie werden aus Ceramiden der Formel (II)-D und/oder (II)-L mit guter Ausbeute und in stereochemisch einheitlicher Form hergestellt. Das Verfahren umfasst den Schutz der 1-Hydroxylgruppe, die Veresterung der 3-Hydroxylgruppe, die Abspaltung der 1-Hydroxylschutzgruppe, die Umsetzung mit dem Trifluor- oder Trichloracetimidat einer 2,3,4,6- tetraacylierten D-Glucose und die Abspaltung der O-Acylgruppen. Bei Verwendung eines D,L-Ceramids (II) wird die Veresterung der 3-Hydroxylgruppe durch eine optisch aktive Säure durchgeführt, gefolgt von der Trennung in die Diastereomeren, oder es wird nach der Umsetzung mit dem D-Glucosederivat eine Trennung in die Diastereomeren vorgenommen.

EP 0 146 810 A2

Croydon Printing Company Ltd.

Bei den bisher bekannten, im menschlichen Organismus vorkommenden Lipiden unterscheidet man zwei Gruppen: die humoralen, nicht strukturgebundenen Lipide und solche, welche Bestandteile von Zellstrukturen sind.

Zu den humoralen Lipiden, welche bei höheren Organismen eine biologische Funktion ausüben, gehören beispielsweise Steroidhormone und Prostaglandine. Letztere spielen bei entzündlichen Reaktionen der Gewebe eine Rolle.

Die strukturgebundenen Lipide spielen, neben der Energiespeicher-Funktion, vor allem in den Zellstrukturen, welche verschiedene Kompartimente der Zellen trennen, eine wichtige Rolle. In zunehmendem Mass wird erkannt, wie einzelne Lipide die Signalübertragung durch diese Membranen, z.B. durch die Veränderung der Membranfluidität (laterale Diffusionsgeschwindigkeit der Membranlipide) beeinflussen und steuern können. Im Verlauf eines Teilungszyklus der Zellen ändern sich die Membranzusammensetzung und damit die physikalischen Eigenschaften kontinuierlich. Als Komponenten dieser Membranen sind vor allem Phospholipide, Sterine und Glykosphingolipide bekannt.

Die Glykosphingolipide sind Abkömmlinge der Ceramide, welche aus einem Aminodiol wie das $C_{18}$-Sphingosin oder das $C_{20}$-Sphingosin:

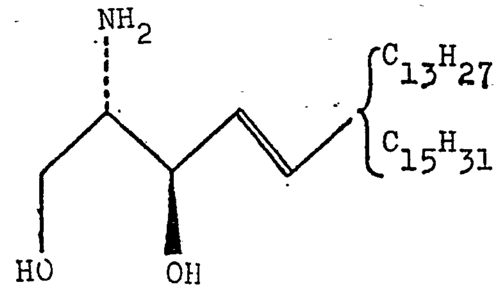

und einem langkettigen Fettsäurerest (RCO-) bestehen und der folgenden allgemeinen Formel entsprechen:

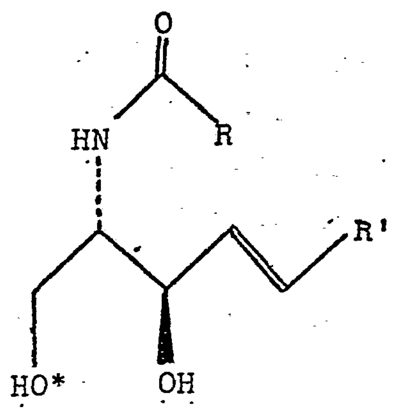

R, R' = langkettige aliphatische Radikale

Ceramide

An die mit * bezeichnete Hydroxylgruppe der Ceramide ist ein Kohlehydratanteil gebunden, welcher aus 1 bis 20 oder sogar mehr Zuckereinheiten bestehen kann.

Je nach der Natur des Kohlehydratanteils fallen die Glykosphingolipide in zwei Hauptklassen. Bei Verknüpfung des Ceramids mit einem oder mehreren Monosacchariden sind es die neutralen Glykosphingolipide - auch Cerebroside genannt , während die Verknüpfung mit einem Oligosaccharid, welches durch Acylneuraminsäuren (auch Sialinsäuren genannt) substituiert ist, die sauren Glykosphingolipide - auch Ganglioside genannt - ergibt. Letzteren werden Rezeptorfunktionen, z.B. für Viren und Toxine zugeschrieben; ferner sollen sie eine neuro-regenerative Wirkung haben.

Dieser Stand der Technik wird insbesondere durch folgende Abhandlungen veranschaulicht:

- K. Jungermann und H. Möhler : Biochemie, Springer-Verlag, Berlin Heidelberg New York 1980, 448-452;
- S. Hakomori, Annual Review of Biochemistry 52 (1981), 733-764.

Bei den eigentlichen, aus dem Hirn stammenden Cerebrosiden besteht die Fettsäurekomponente meistens aus einer $C_{24}$-Carbonsäure, welche eine Hydroxylgruppe in α-Stellung oder eine Doppelbindung tragen kann. Beispielsweise findet sich im Kerasin die Lignocerinsäure $C_{24}H_{48}O_2$, im Nervon die Nervonsäure $C_{24}H_{46}O_2$, im Cerebron oder Phrenosin die Cerebronsäure $C_{24}H_{48}O_3$ und im Hydroxynervon die Hydroxynervonsäure $C_{24}H_{46}O_3$. In diesen und in den meisten Cerebrosiden besteht der Kohlehydratanteil aus 1 Mol Galaktose.

In neuerer Zeit sind weitere neutrale Glykosphingolipide entdeckt worden, welche eine Fettsäurekomponente mit kürzerer aliphatischer Kette und einen aus mehreren Zuckereinheiten bestehenden Kohlehydratanteil aufweisen. Diese Verbindungen sind aber nicht im Gehirn, sondern in anderen Organen, beispielsweise im Darm, in der Milz, in der Leber und in den Erythrozyten, gefunden worden, weshalb der Name Cerebroside nicht mehr oder nur für die zuvor erwähnte Gruppe verwendet werden sollte.

Ueberraschenderweise wurde nun eine neue Gruppe der neutralen Glykosphingolipide gefunden, welche sich gegenüber den oben erwähnten, bereits bekannten Verbindungen durch eine andere Fettsäurekomponente und/oder einen anderen Kohlehydratanteil, nämlich 1 Mol D-Glukose, auszeichnen. Diese neue chemische Struktur wiederspiegelt sich in einer bei den Glykosphingolipiden bisher ebenfalls unbekannten wundheilungsfördernden bzw. einer zell- und geweberegenerierenden Wirkung .

Diese Verbindungen werden durch die Formel (I)-D und (I)-L auf beiliegendem Formelblatt wiedergegeben; wie aus den Formeln hervorgeht, besitzen sie die erythro-Konfiguration.

Sofern es sich um Verbindungen der Formel (I)-D handelt, können sie als Zwischenprodukte in der Biosynthese bzw. im Metabolismus von in der Natur (Organe und Körperflüssigkeiten von Säugetieren) vorkommenden höheren Glykosphingolipiden, wie die Ganglioside, welche einen aus mehreren Zuckereinheiten bestehenden Kohlehydratanteil aufweisen, aufgefasst werden. Die Verbindungen der Formel (I)-L hingegen haben in der Natur keinen Platz und können auch nicht als Teilformel oder Vorläufer von komplexeren Verbindungen aufgefasst werden.

In den Formeln (I)-D und (I)-L bedeuten:
$R^1$ den Acylrest einer Fettsäure mit 14 bis 24 Kohlenstoffatomen oder die entsprechenden Acylreste mit einer Hydroxylgruppe in α-Stellung oder mit einer oder zwei Doppelbindungen in cis-Konfiguration und
$R^2$ den Pentadecanyl- oder Heptadecanylrest oder die entsprechenden $C_{15}$- und $C_{17}$-Reste mit einer, zwei oder drei Doppelbindungen, von welchen jeweils eine in 1,2-Stellung sitzt und trans-Konfiguration aufweist, die andere oder anderen, wenn vorhanden, cis-Konfiguration aufweisen.

Die chemische Struktur ist im Kohlehydratanteil homogen; dennoch ist die Vielfalt der wundheilungsfördernden Glukosphingolipide bemerkenswert. Dabei ist jedoch zu beachten, dass diese Vielfalt alleine durch Variation des Lipidanteils zustandekommt. Das erscheint biologisch sinnvoll, denn hauptsächlich der aus einer Membran nach aussen gerichtet Kohlehydratanteil des Moleküls ist für die biologische Aktivität bestimmend. Die Fluidität von Membranen wird z.B. von Glukosphingolipiden beeinflusst, indem das Amidproton des Ceramids mit den Phosphatgruppen von Phospholipiden in Wechselwirkung treten kann und somit einer Lipidmembran mehr Stabilität verleiht als sie nur durch eine Phospholipid/Steroid-Wechselwirkung allein zustande kommen kann.

Die Verbindungen entfalten in vivo eine fördernde Wirkung auf die Zell- und Geweberegeneration. Diese Wirkung lässt sich auch in vitro, mit Hilfe von Zellkulturen, nachweisen. Wird nämlich in einer Zellkultur, beispielsweise eine Fibroblastenkultur, vorerst die Teilungsrate durch Einwirkung eines schädigenden Agens künstlich herabgesetzt und wird die Kultur danach mit den Verbindungen behandelt, so wird die Teilungsrate in kurzer Zeit wieder auf einen normalen, mit jenem einer gesunden, unbeschädigten Kultur vergleichbaren oder identischen Wert gebracht. Dieselbe Behandlung einer parallel geführten, aber gesunden Zellkultur bewirkt hingegen keine Veränderung der Teilungsrate. Es handelt sich also dabei nicht etwa um eine blosse mitotische Wirkung.

Infolge der beschriebenen fördernden Wirkung auf die Regenerationsmechanismen geschädigter Zellen eignen sich die Verbindungen für eine therapeutische Anwendung bei Wunden jeglicher Genese, insbesondere bei schlecht oder langsam heilenden Wunden oder Ulzerationen. In der Tat führen sie insbesondere bei topischer Anwendung auf Wunden, wie Ulcus cruris, Geschwüre des Magendarmtraktes, insbesondere Ulcus ventriculi und Ulcus duodeni, diabetische Gangrän, Strahlenschäden, Verbrennungen und Hauttransplantationen, zur Bildung von gesundem, gut durchblutetem neuem Gewebe ohne störende Narben.

Infolge ihrer höheren therapeutischen Wirksamkeit werden die Sphingosinderivate der Formel (I)-D ganz allgemein bevorzugt.

Andererseits werden wegen ihrer höheren spezifischen Aktivität (Aktivität per Mikrogramm der Verbindung) jene Sphingosinderivate der Formel (I)-D und (I)-L ebenfalls bevorzugt, bei welchen $R^1$ den Acylrest einer Fettsäure mit 14 bis 20 Kohlenstoffatomen oder die entsprechenden Acylreste mit einer Hydroxylgruppe in $\alpha$-Stellung oder mit einer oder zwei Doppelbindungen in cis-Konfiguration bedeutet, $R^2$ jedoch die oben angegebene Bedeutung beibehält.

Besonders bevorzugt wird die engere Gruppe von Sphingosinderivaten, welche durch die Formel (I)-D wiedergegeben wird, in welcher $R^1$ den Acylrest einer Fettsäure, einer α-Hydroxyfettsäure oder einer einfach oder zweifach cis-olefinisch ungesättigten Fettsäure mit 14 bis 20 Kohlenstoffatomen bedeutet und $R^2$ die oben angegebene Bedeutung beibehält.

Beispiele der erfindungsgemässen Verbindungen sind u.a.:
das D- und das L-erythro-1-(β-D-Glucopyranosyloxy)-3-hydroxy-2-myristoylamino-4-trans-octadecen und die entsprechenden -4-trans-eicosene;
das D- und das L-erythro-1-(β-D-Glucopyranosyloxy)-3-hydroxy-2-palmitoylamino-4-trans-octadecen und die entsprechenden -4-trans-eicosene;
das D- und das L-erythro-1-(β-D-Glucopyranosyloxy)-3-hydroxy-2-stearoylamino-4-trans-octadecen und die entsprechenden -4-trans-eicosene;
das D- und das L-erythro-1-(β-D-Glucopyranosyloxy)-3-hydroxy-2-(9-cis-octadecenoylamino)-4-trans-octadecen und die entsprechenden -4-trans-eicosene;
das D- und das L-erythro-1-(β-D-Glucopyranosyloxy)-3-hydroxy-2-(cis,cis-9,12-octadecadienoylamino)-4-trans-octadecen und die entsprechenden -4-trans-eicosene;
das D- und das L-erythro-1-(β-D-Glucopyranosyloxy)-3-hydroxy-2-eicosanoylamino-4-trans-octadecen und die entsprechenden -4-trans-eicosene;
das D- und das L-erythro-1-(β-D-Glucopyranosyloxy)-3-hydroxy-2-docosanoylamino-4-trans-octadecen und die entsprechenden -4-trans-eicosene;
das D- und das L-erythro-1-(β-D-Glucopyranosyloxy)-3-hydroxy-2-tetracosanoylamino-4-trans-octadecen und die entsprechenden -4-trans-eicosene.

Erfindungsgemäss werden die Sphingosinderivate der Formel (I)-D oder (I)-L in reinem Zustande und mit befriedigender Ausbeute durch Totalsynthese hergestellt. Das Verfahren eignet sich vorzüglich zur Anwendung in industriellem Massstabe und macht die Herstellung der Verbindungen von jeglicher natürlicher Quelle unabhängig.

Das Verfahren ist dadurch gekennzeichnet, dass man eine optisch aktive Verbindung der Formel (II)-D oder (II)-L, in welcher $R^1$ und $R^2$ die oben angegebenen Bedeutungen haben, oder das entsprechende Racemat mit einem organischen Reagens, das mit einer primären Hydroxylgruppe selektiv zu reagieren vermag, unter Bildung von Verbindungen der Formel (III), in welchen R eine Hydroxylschutzgruppe bedeutet, umsetzt,

(A) die Verbindung der Formel (III) mit einer organischen Carbonsäure unter Bildung einer Verbindung der Formel (IV), in welcher $Ac^1$ den Acylrest einer organischen Carbonsäure bedeutet, verestert oder

(B) bei Verwendung eines Racemates als Ausgangsprodukt die Verbindung der Formel (III) mit einer optisch aktiven organischen Säure verestert und das erhaltene Gemisch von diastereomeren Verbindungen der Formel (V), in welchen $Ac^2$ den Acylrest einer optisch aktiven organischen Säure bedeutet, in die Diastereomeren trennt oder

(C) auf die Variante (B) anschliessend, die einzelnen Diastereomeren der Formel (V) desacyliert und mit einer organischen Carbonsäure unter Bildung von enantiomeren Verbindungen der Formel (VI), in welchen $Ac^3$ den Acylrest einer organischen Carbonsäure bedeutet, verestert,

die bei der Variante (A) erhaltene Verbindung der Formel (IV) bzw.

die bei der Variante (B) erhaltenen Diastereomeren der Formel (V) bzw.

die bei der Variante (C) erhaltenen Enantiomeren der Formel (VI) von der Hydroxylschutzgruppe R unter Bildung entsprechender Verbindungen der Formel (VII), (VIII) bzw. (IX) befreit, die Verbindung der Formel (VII),

(VIII) bzw. (IX) mit dem O-Trifluor- oder O-Trichloracetimidat einer D-Glucose, deren Hydroxylgruppen in den 2-, 3-, 4- und 6-Stellungen durch Acylreste $Ac^4$ geschützt sind, unter Bildung von Verbindungen der jeweils entsprechenden Formel (X), (XI) bzw. (XII) umsetzt, wenn für die Variante (A) als Ausgangsprodukt ein Racemat verwendet wird, die Verbindung der Formel (X) in die Diastereomeren trennt, und von den Verbindungen der Formel (X), (XI) bzw. (XII) die Acylgruppen $Ac^1$, $Ac^2$, $Ac^3$ und $Ac^4$ gleichzeitig abspaltet, wobei aus Verbindungen der D- bzw. der L-Reihe jeweils Verbindungen der D- bzw. der L-Reihe entstehen.

Ausgangsprodukte des Verfahrens sind Ceramide der Formel (II)-D und/oder (II)-L; das Verfahren kann nämlich sowohl auf eine der optisch aktiven Verbindungen als auch auf das entsprechende Racemat angewendet werden. Wird als Ausgangsprodukt ein Racemat eingesetzt, so erfolgt auf einer bestimmten Verfahrensstufe eine Trennung in Diastereomere bzw. in Enantiomere, so dass als Endprodukt in jedem Fall die reinen und stereochemisch einheitlichen Verbindungen der Formel (I)-D oder (I)-L erhalten werden.

Ihrerseits können die Ceramide der Formel (II)-D bzw. (II)-L oder ihr Racemat aus entsprechenden $C_{18}$- oder $C_{20}$-Sphingosinen durch N-Acylierung mittels einer Fettsäure der Formel $R^1$-OH, in welcher $R^1$ die oben angegebene Bedeutung hat, oder eines reaktionsfähigen funktionellen Derivates derselben hergestellt werden.

Die Fettsäure $R^1OH$ ist beispielsweise die Myristinsäure, die Palmitinsäure, die Stearinsäure, die

Oelsäure (cis-9-Octadecensäure), die Linolsäure (cis, cis-9,12-Octadecadiensäure), die Arachinsäure (Eicosansäure), die Behensäure (Docosansäure) oder - an der oberen Grenze der für $R^1$ angegebenen Bedeutung - die Tetracosansäure (Lignocerinsäure), die cis-15-Tetracosensäure (Nervonsäure), die 2-Hydroxytetracosansäure (Cerebronsäure), die 2-Hydroxy-15-tetracosensäure (Hydroxynervonsäure) oder die mit letzterer isomere 2-Hydroxy-17-tetracosensäure.

Die Acylierung mit der Fettsäure $R^1$-OH kann nach dem Verfahren von D. Shapiro und Mitarb. [J. Am. Chem. Soc. 81, 4360 (1959)] bewerkstelligt werden; eine besonders ergiebige Arbeitsweise ist dafür entwickelt worden, wie im experimentellen Teil beschrieben wird.

Die den natürlich vorkommenden Glykosphingolipiden, Cerebrosiden und Gangliosiden zugrundeliegenden Sphingosine besitzen die erythro-Konfiguration und gehören der D-Reihe an. Das erfindungsgemässe Verfahren bringt nun die Möglichkeit, auch racemische Sphingosine in die Endprodukte der Formel (I)-D und (I)-L zu überführen. Dies ist von besonderer Bedeutung, weil die bekannten Synthesen der D-Sphingosine über zahlreiche, mitunter heikle Verfahrensstufen verlaufen, während eine neuere, einfache Synthese von R.R. Schmidt und R. Kläger [Angew. Chem. 94, 215-216 (1982); Angew. Chem. Int. Ed. Engl. 21, 210-211 (1982); Angew. Chem. Suppl. 1982, 393-397] die racemischen Sphingosine mit guter Ausbeute liefert.

Im folgenden wird das Verfahren ausführlicher beschrieben.

Der Schutz der primären Hydroxylgruppe der Ceramide (II)-D und/oder (II)-L soll mit Reagenzien vorgenommen werden, welche in Gegenwart einer primären und einer sekundären Hydroxylgruppe selektiv mit der ersteren reagieren. Es eignen sich als Schutzgruppe R insbesondere solche, die eine grosse räumliche Beanspruchung aufweisen wie die tert.Butyl-, Triphenylmethyl- (Trityl-), Trichloracetyl-, Trimethylsilyl-, tert.Butyldimethylsilyl- oder tert.Butyldiphenylsilylgruppe. Bevorzugt werden die Triphenylmethyl-, Monomethoxytriphenylmethyl-, tert.Butyldimethylsilyl- und tert.Butyldiphenylsilylgruppe.

Die Einführung der Schutzgruppe R erfolgt nach den bekannten Methoden der organischen Chemie, entsprechend der Art der gewählten Schutzgruppe. Beispielsweise kann die Triphenylmethylgruppe durch Behandeln des Ceramids mit einem entsprechenden Halogenid wie das Triphenylchlormethan oder das Triphenylbrommethan eingeführt werden. Auch für die tert.Butyldimethylsilyl- und die tert.Butyldiphenylsilylgruppe kann das entsprechende Halogenid, vorzugsweise das Chlorid oder das Bromid, mit Vorteil benützt werden.

Gemäss einer ersten Verfahrensvariante (A) wird nun das in 1-Stellung geschützte Ceramid der Formel (III)-D und/oder (III)-L in der 3-Stellung mit einer organischen Carbonsäure der Formel $Ac^1OH$ oder einem reaktionsfähigen funktionellen Derivat derselben zu Verbindungen der Formel (IV)-D und/oder (IV)-L verestert. Es eignen sich dazu vor allem aliphatische und aromatische Carbonsäuren; bevorzugt wird die Verwendung einer monocyclischen aromatischen Carbonsäure wie die Benzoesäure oder eine substituierte Benzoesäure. Dieselben Carbonsäuren eignen sich ebenfalls bzw. werden ebenfalls bevorzugt bei der unten besprochenen Veresterung mit der Säure der Formel $Ac^3OH$.

Die Veresterung, ob mit der Carbonsäure $Ac^1OH$ bzw. $Ac^3OH$ oder mit der optisch aktiven organischen Säure $Ac^2OH$, kann nach den in "Ullmanns Encyklopädie der technischen Chemie", 4. Auflage, Band 11, Seiten 91 und folg., Verlag Chemie, Weinheim BRD (1976), beschriebenen Methoden durchgeführt werden. Sie erfolgt mit Vorteil unter Benutzung eines Carbonsäurehalogenids in Gegenwart einer tertiären organischen Base wie Triethylamin, Pyridin oder Dimethylanilin, in einem wasserfreien organischen Lösungsmittel wie Benzol, Toluol, Tetrahydrofuran, Diethylether oder Dichlormethan.

Gemäss einer zweiten Verfahrensvariante (B) wird das in 1-Stellung geschützte Ceramid der Formel (III) in der 3-Stellung mit einer einfachen optisch aktiven organischen Carbonsäure $Ac^2OH$ oder einem reaktionsfähigen funktionellen Derivat derselben zu Verbindungen der Formel (V) verestert. Es eignen sich dazu die Weinsäure, die Dibenzoylweinsäure, die Mandelsäure, die O-Acetylmandelsäure, die Camphersäure, die Camphersulfonsäure oder die Bromcamphersulfonsäure usw; bevorzugt wird die O-Acetylmandelsäure. Die Veresterung erfolgt mit Vorteil unter Benutzung eines Carbonsäurehalogenids in Gegenwart einer tertiären organischen Base, wie Pyridin, in einem wasserfreien organischen Lösungsmittel wie Benzol, Toluol, Tetrahydrofuran, Diethylether oder Dichlormethan.

Anschliessend erfolgt eine Auftrennung des

Gemisches diastereomerer Verbindungen der Formel (V) durch Chromatographie, vorzugsweise auf Kieselgel, oder durch fraktionierte Kristallisation.

Gemäss einer dritten Verfahrensvariante (C) werden die nach (B) erhaltenen Diastereomeren der Formel (V) durch basenkatalysierte Abspaltung der 3-O-Acylgruppe ($Ac^2$), vorzugsweise in Natriummethanolat/Methanol, in die optischen Antipoden übergeführt. Diese werden dann jeweils mit einer einfachen organischen Carbonsäure der Formel $Ac^3OH$ oder einem reaktionsfähigen Derivat derselben zu Verbindungen der Formel (VI) verestert. Es eignen sich dazu einfache aromatische und aliphatische Carbonsäuren. Bevorzugt wird die Verwendung einer aromatischen Carbonsäure, beispielsweise der Benzoesäure oder einer substituierten Benzoesäure.

Die bei den Varianten (A), (B) und (C) erhaltenen Verbindungen (IV), (V) und (VI) werden zur Abspaltung der Schutzgruppe in 1-Stellung einer sauren Hydrolyse (Tritylschutzgruppen, Silylschutzgruppen) unterworfen und so die Verbindungen (VII), (VIII) und (IX) erhalten.

Die Verbindungen (VII), (VIII) und (IX) werden mit dem O-Trichlor- oder O-Trifluoracetimidat einer D-Glucose, deren Hydroxylgruppen ausser jener an der 1-Stellung durch Acylreste $Ac^4$ geschützt sind, unter Bildung von Verbindungen der Formel (X), (XI) und (XII) umgesetzt. Im Fall der Verbindung (X) wird die Auftrennung in die Diastereomeren durchgeführt und von den Verbindungen (X), (XI) und (XII) werden die Acylgruppen $Ac^1$, $Ac^2$, $Ac^3$ und $Ac^4$ gleichzeitig basenkatalysiert, vorzugsweise in Natriummethanolat/Methanol, abgespalten.

Beschreibung der pharmakologischen Versuche

Versuch 1

Ratten werden narkotisiert und die Fellhaare werden ihnen entfernt. Durch flaches Auflegen einer Metallscheibe von 2 cm Durchmesser und einer Temperatur von 270 °C während 17 Sekunden wird beidseitig am Rumpf eine Verbrennungswunde verursacht. Die Glukosphingolipide werden in eine Geléegrundlage eingearbeitet und diese wird täglich zweimal auf die Wunde gestrichen. Es wird die Dauer bis zur Endabheilung der Wunden gemessen. Gelées, welche die Glukosphingolipide enthalten, ergeben gegenüber der Kontrollgruppe eine Verkürzung der Abheilungsdauer um bis zu 21 %.

Versuch 2

An Minipigs werden dorsal je vier Verbrennungswunden wie in Versuch 1 beschrieben und mit einem Hohlzylinder-Bohrer 4 Stanzwunden mit einem Durchmesser von 2,5 cm gesetzt. Die Wirkstoffe werden in eine Geléegrundlage eingerarbeitet und die Wunden werden zweimal täglich mit dem Gelée bestrichen. Die Dauer bis zur Endabheilung wird festgehalten. Die Dauer bis zur Endabheilung wird durch die Glukosphingolipide um bis zu 18 % verkürzt.

Versuch 3

An Minipigs werden, wie in Versuch 1 beschrieben, Verbrennungswunden gesetzt. Nach 6, 12, 18 und 22 Tagen täglicher Wundbehandlung werden aus der Behandlungsgruppe Tiere entfernt und in Narkose getötet. Die Wunden werden ausgeschnitten, halbiert und in 4 % gepuffertem Formalin fixiert. Diese Gewebestücke werden zu 4 µm dicken histologischen Paraffinschnitten verarbeitet. Folgende Parameter werden quantitativ erfasst:

20.11.84

1. Länge der epithelialisierten Wundoberfläche
2. Länge der nicht-epithelialisierten Wundoberfläche
3. Länge des Stratum basale der Epidermis
4. Fläche der neugebildeten Epidermis
5. Fläche von Haarfollikeln und Talgdrüsen

Die Auswertung der Parameter ergibt, dass die mit Glukosphingolipid-haltigem Gelée behandelten Tiere eine längere epithelialisierte Wundoberfläche und eine kürzere nichtepithelialisierte Wundoberfläche haben als die mit einer wirkstofflosen Geléegrundlage behandelten Tiere.

Versuch 4

An narkotisierten Ratten werden 1 cm breite und 5 mm tiefe Stanzwunden gesetzt. In diese Wundlöcher werden Viskose-Zellulose-Hohlzylinder-Schwämme eingesetzt. In die innere Aushöhlung der Hohlzylinder werden täglich 100 µl einer glukosphingolipid-haltigen Lösung mit einem Gehalt an Glukosphingolipiden von 0,1 bis 15 µg/ml eingespritzt. 16 und 24 Tage nach der Implantation werden die Schwämme entnommen und auf den Gehalt an Hämoglobin, Desoxyribonukleinsäure und Hydroxyprolin untersucht. Die Wunden, welche mit

den Glukosphingolipiden behandelt worden sind, haben einen signifikant höheren Hämoglobin-, DNS- und Hydroxyprolin-Gehalt in den Schwämmen als jene der Kontrolltiere.

Versuch 5

Fibroblasten-Zellkulturen, welche in einem Nährmedium, das mit Bicarbonat und $CO_2$-haltiger Atmosphäre bei pH 7,2 gepuffert ist, gewachsen sind, werden einem neuen Nährmedium ausgesetzt, das kein Bicarbonat enthält und das normaler Atmosphäre ausgesetzt ist, wobei der pH 7,2 durch Zugabe eines geeigneten, nicht-toxischen Puffers, wie 2-[4-(2-Hydroxyäthyl)-piperazin-1-yl]-äthansulfonsäure/NaOH-Lösung (HEPES), stabilisiert wird. Zellen, welche im Nährmedium kein Glukosphingolipid zur Verfügung haben, stellen das Wachstum nahezu ein, während sich Zellen in wirkstoffhaltigem Medium rasch erholen und die gleiche Wachstumsrate erlangen, wie Kontrollkulturen in bicarbonathaltigem Medium.
Gemessen wird die Wachstumsrate z.B. nach dreitägiger Glukosphingolipid-Wirkung, indem den Zellen während 5 Stunden $^3H$-Thymidin angeboten wird. Die Zellen werden danach durch osmotischen Schock aufgeschlossen und die DNS wird auf einem Diäthylaminoäthyl-Filterpapier zurückgehalten. Die Radioaktivität dieser Filter wird gemessen.

Die folgenden Beispiele veranschaulichen bevorzugte Ausführungsformen der Erfindung.

$^1$H-NMR-Spektren wurden mit dem 250 MHz-Gerät WM 250 Cryospec der Firma Bruker, Spectrospin, Industriestrasse 26, CH-8117 Fällanden/Zürich, gemessen. Die Verschiebungen sind auf Tetramethylsilan (TMS) als internen Standart bezogen und in ppm angegeben.

Die angegebenen Schmelzpunkte wurden in einem Kupferblock bestimmt und sind nicht korrigiert.

Zur analytischen Dünnschichtchromatographie (DC) wurden Kieselgelplatten der Firma E. Merck AG, Darmstatt (BRD), verwendet. Die Dünnschichtchromatogramme wurden, sofern die Substanzen nicht UV-aktiv waren, mit 15 % Schwefelsäure besprüht und bei 120°C entwickelt.

Präparative Säulenchromatographien wurden mit Kieselgel 60 0,062-0,200 mm der Firma Merck durchgeführt. Für die Mitteldruckchromatographie wurden Fertigsäulen nach D. Flockerzi, Diplomarbeit, Universität Stuttgart (1978), mit Kieselgel "LiChroprep Si 60,15-25" verwendet.

Die Ausbeuten wurden auf der Reinigungsstufe angegeben, auf der NMR-spektroskopisch und mittels Dünnschichtchromatographie keine Verunreinigungen nachzuweisen waren.

20.11.84

Herstellung der Ausgangsprodukte

(a) Tetradecanal

32,3 g (150 mMol) Pyridiniumchlorochromat werden bei Raumtemperatur unter starkem Rühren in 200 ml wasserfreiem Methylenchlorid suspendiert und mit einer Lösung von 21,4 g (100 mMol) Tetradecanol in 20 ml wasserfreiem Methylenchlorid versetzt. Das Reaktionsgemisch kommt hierbei zum Sieden. Nach 1 1/2 Std. ist die Reaktion beendet. Man dekantiert ab und wäscht mit ca. 200 ml trockenem Diethylether nach. Nach Filtrieren und Einengen des Lösungsmittels wird über Kieselgel mit Petroleumbenzin (Sdp. 35-80°C)/Essigester 9:1 chromatographiert. Ausbeute: 18 g (84 %).

(b) Hexadecanal

21,5 g (100 mMol) Pyridiniumchlorochromat werden in 200 ml wasserfreiem Dichlormethan suspendiert. 16 g (66 mMol) Hexadecanol in 50 ml wasserfreiem Dichlormethan werden dazugetropft. Nach 2 Std. verdünnt man mit 300 ml wasserfreiem Ether und dekantiert vom schwarzen Rückstand ab. Der Rückstand wird dreimal mit ca. 50 ml wasserfreiem Ether gewaschen. Die organischen Phasen werden vereinigt und der Ether wird bis zur Trockene eingedampft. Es wird über Kieselgel mit Petrolether/Essigester 9:1 chromatographiert. Ausbeute: 15 g (95 %). Smp. 33-34°C.

(c) Formylmethylentriphenylphosphoran

Die Verbindung wird nach dem Verfahren gemäss J. Chem. Soc. 1961, 1266-1272 hergestellt.

(d) 2-trans-Hexadecenal

100 g (0,47 Mol) Tetradecenal und 173 g (0,56 Mol) Formylmethylentriphenylphosphoran werden in 1 Liter wasserfreiem Chloroform 12 Std. zum Sieden erhitzt. Nach Abkühlen wird eingeengt und über eine kurze Kieselgelsäule mit Petroleumbenzin (Sdp. 35-80°C)/ Essigester 9:1 chromatographiert, um das Triphenylphosphinoxid zu entfernen. Anschliessend wird im Hochvakuum destilliert. Die Verbindung erweist sich mit jener gemäss Hoppe-Seyler's Z. Physiol. Chem. 354, 1626-1632 (1973) identisch. Ausbeute: 77 g (69 %).

$R_F$ = 0,5, Petroleumbenzin (Sdp. 35-80°C)/Essigester 9:1; UV: Blaufärbung mit Anisaldehydreagenz (0,5 ml Anisaldehyd, 50 ml Eisessig, 1 ml $H_2SO_4$); Sdp. ($10^{-3}$ Torr): 115°C.

(e) 2-trans-Octadecenal

12 g (50 mMol) Hexadecenal und 15,2 g (50 mMol) Formylmethylentriphenylphosphoran werden in 250 ml wasserfreiem Toluol 8 Std. lang am Rückfluss gekocht. Es wird bis zur Trockene eingedampft. Der Rückstand wird fünfmal mit 100 ml Ether extrahiert. Der Extrakt wird bis zur Trockene eingedampft und mit Toluol über Kieselgel chromatographiert. Ausbeute: 8,2 g (62 %). Smp.: 35°C; $R_F$ = 0,8, Toluol/Essigester 8:2.

(f) D,L-erythro-2-Amino-1,3-dihydroxy-4-trans-octadecen ($D,L-C_{18}$-Sphingosin)

Diese Verbindung wird nach dem Verfahren gemäss Angew. Chem. 94, 215-216 (1982); Angew. Chem. Int. Ed. Engl. 21, 210-211 (1982); Angew. Chem. Suppl. 1982, 393-397 hergestellt.

(g) D,L-erythro-2-Amino-3-hydroxy-4-trans-eicosadecen-säure

Die Verbindung wurde nach demselben Verfahren wie bei Verbindung (f) angegeben dargestellt.

1,1 ml (7,9 mMol) trockenes Diisopropylamin wird unter Stickstoff zu einer auf -40°C abgekühlten Lösung von 5,8 mMol n-Butyllithium in 30 ml wasserfreiem, Stickstoff-gesättigtem Tetrahydrofruan gegeben. Es wird 30 Min. bei dieser Temperatur gerührt und dann auf -80°C abgekühlt. Man lässt 1,6 g (5,6 mMol) N,N-Bis-(trimethylsilyl)-glycin-trimethylsilyl-ester [Angew. Chem. Int. Ed. Engl. 80, 797 (1968)] in wenig Tetrahydrofuran gelöst langsam zutropfen, wobei sich die Lösung gelb bis braun färbt. Nach 90 Min. werden 2,2 g (8,4 mMol) 2-trans-Octadecenal gelöst in Tetrahydrofuran dazugetropft. Man lässt weitere 90 Min. bei -80°C rühren. Anschliessend erwärmt man auf Raumtemperatur und säuert mit gesättigter ethanolischer Salzsäure auf pH 5 an. Nach dem Absaugen wäscht man mit Wasser das Diisopropylaminhydrochlorid und das Glycinhydrochlorid aus. Ausbeute: 1,6 g (88 %). Smp.: 150°C (Zersetzung).

Elementaranalyse für $C_{20}H_{39}NO_3$ (341,49):

| | C | H | N |
|---|---|---|---|
| berechnet: | 70,34 | 11,49 | 4,10 |
| gefunden : | 70,56 | 11,62 | 4,06 |

$^1$H-NMR (in DMSO): 5,75-5,62 (m, 1H, -C<u>H</u>=CH-CHOH); 5,42-5,30 (dd, 1H, C=C<u>H</u>-CHOH, $J_{trans}$= 15,5 Hz, $J_{vic}$= 6,4 Hz); 4,28-4,20 (dd, 1H, -C=CH-C<u>H</u>OH, $J_1$= $J_2$= 6,1 Hz); 3,50-3,20 (m, OH, $NH_3$, $H_2O$); 3,17-3,12 (d, 1H, -C<u>H</u>-COOH, J= 6.1Hz); 2,03-1,91 (m, 2H, $C\underline{H}_2$-C=C); 1,40-1.10 (m, 26H, aliphat.); 0,90-0,80 (t, 3H, $-CH_3$).

(h) D,L-erythro-2-Amino-1,3-dihydroxy-4-trans-eicosen (D,L-$C_{20}$-Sphingosin)

5,2 g (15,2 mMol) Verbindung (g) werden in 500 ml wasserfreiem Tetrahydrofuran suspendiert. Man gibt 4,1 g (107 mMol) Lithiumaluminiumhydrid in kleinen Portionen hinzu. Es wird 36 Std. am Rückfluss zum Sieden erhitzt. Ueberschüssiges $LiAlH_4$ wird durch vorsichtiges Zutropfen von Wasser zerstört. Nach Zutropfen von 30 ml 2 N Natronlauge kann ein gut filtrierbarer Niederschlag erhalten werden. Man filtriert, wäscht mit Tetrahydrofuran nach und engt bis zur Trockene ein. Zurück bleibt eine leicht gelbliche, wachsartige Substanz. Ausbeute: 3,8 g (76 %). Smp.: 58-60°C; $R_F$ = 0,2, Chloroform/Methanol 1:1.

$^1$H-NMR (in $CDCl_3$):

5,82-5,71 (m, 1H, -C$\underline{H}$=CH-CHOH); 5,51-5,43 (dd, 1H, -CH=C$\underline{H}$-CHOH, $J_{trans}$= 15,5 Hz $J_{vic}$= 7,3 Hz); 4,09-4,02 (dd, 1H,C=CH-C$\underline{H}$OH, $J_1$= $J_2$= 6,1 Hz); 3,75-3,59 (m, 2H,-C$\underline{H}_2$OH); 2,94-2,85 (m, 1H, -C$\underline{H}$-$NH_2$); 2,49-2,15 (m, 3H, -N$\underline{H}_2$, $\underline{H}$O-CH-); 2,14-1,97 (m, 3H, -$CH_2$-O$\underline{H}$, C=CH-$CH_2$); 1,45-1,12 (m, 26H, aliphat.) 0,95-0,82 (t, 3H, -$CH_3$).

Allgemeine Vorschrift zur Synthese der Ceramide

2 g (6,1 mMol) Sphingosin werden in 100 ml Tetrahydrofuran gelöst. Man lässt gleichzeitig 50 ml 50 % wässrige Natriumacetatlösung und 7,3 mMol des jeweiligen Fettsäurechlorides, gelöst in 20 ml wasserfreiem Ether, zutropfen. Man rührt 2 Std. bei Raumtemperatur. Die Etherphase wird abgetrennt und zweimal mit je 50 ml wässriger Natriumhydrogencarbonatlösung und mit 50 ml Wasser ausgeschüttelt. Die organische Phase wird bis zur Trockene eingeengt. Der Rückstand wird aus 100 ml Methanol und einmal aus 100 ml n-Hexan umkristallisiert.

(i) D,L-erythro-1,3-Dihydroxy-2-palmitoylamino-4-trans-octadecen

8 g (30 mMol) Sphingosin werden in 200 ml Tetrahydrofuran gelöst und unter intensivem Rühren mit 100 ml einer 50 % Natriumacetatlösung versetzt. Dazu werden 11 g (40 mMol) Palmitoylchlorid, gelöst in 15 ml wasserfreiem Ether, langsam zugetropft. Anschliessend lässt man noch 1 Std. bei Raumtemperatur rühren. Die

wässrige Phase wird abgetrennt und die organische Phase wird mehrmals mit gesättigter Natriumhydrogencarbonatlösung gewaschen. Nach Trocknen über Natriumsulfat und Einengen wird aus Methanol umkristallisiert. Ausbeute: 10 g (70 %). Smp. 94-96°C.

$R_F$ = 0,57, Chloroform/Methanol 9:1 (D,L-Sphingosin: $R_F$ = 0,04); $^1$H-NMR (80 MHz, $CDCl_3$ in ppm): 7,0 (brd, 1H, NH); 5,75 (m, 2H, HC=CH), 4,3 (m, 1H,-<u>CH</u>-N); 4,1-3,6 (m, 5H).

(j) D,L-erythro-1,3-Dihydroxy-2-palmitoylamino-4-trans-eicosen

Durchführung der Synthese nach der allgemeinen Vorschrift. Ausbeute: 2,66 g (77 %). Smp. 88-89°C; $R_F$ = 0,15, Dichlormethan/Methanol 95:5.

Elementaranalyse für $C_{36}H_{71}NO_3$ (565,89):

| | C | H | N |
|---|---|---|---|
| berechnet: | 76,41 | 12,63 | 2,47 |
| gefunden : | 76,22 | 12,61 | 2,47 |

$^1$H-NMR (in $CDCl_3$):

6,28-6,19 (d, 1H, N<u>H</u>, J= 8,8 Hz); 5,88-5,71 (m, 1H, C<u>H</u>-CH-CHOH); 5,60-5,47 (dd, 1H, HC=C<u>H</u>-CHOH, $J_{trans}$= 15,5 Hz $J_{vic}$=6,7 Hz); 4,39-4,29 (m, 1H, C<u>H</u>-NH); 4,02-3,88 (m, 2H, C=C-C<u>H</u>OH, C<u>H</u>$_2$OH); 3,78-3,67 (m, 1H, C<u>H</u>$_2$OH); 2,68-2,55 (m, 1H, O<u>H</u>); 2,29-2,19 (t, 2H, CO-C<u>H</u>$_2$, J= 7,6 Hz); 2,12-2,01 (m, 2H, C=C-C<u>H</u>$_2$); 1,70-1,61 (m, 2H, CO-$CH_2$-C<u>H</u>$_2$); 1,45-1,15 (m, 50H, aliphat); 0,95-0,83 (t, 6H, $CH_3$).

(k) D,L-erythro-1,3-Dihydroxy-2-stearoylamino-4-trans-eicosen

Durchführung der Synthese nach der allgemeinen Vorschrift. Ausbeute: 2,42 g (67 %). Smp.: 90-91°C; $R_F$ = 0,15, Dichlormethan/Methanol 95:5.

Elementaranalyse für $C_{38}H_{75}NO_3$ (595,95):

| | C | H | N |
|---|---|---|---|
| berechnet: | C 76,84 | H 12,71 | N 2,36 |
| gefunden : | 76,64 | 12,73 | 2,33 |

$^1$H-NMR (in $CDCl_3$): 6.28-6,19 (d, 1H, NH, J= 8,8 Hz); (m, 1H, CH CH-CHOH); 5,60-5,47 (dd, 1H, C=CH-CHOH, $J_{trans}$=15,5 Hz, $J_{vic}$= 6,7 Hz); 4,37-4,28 (m, 1H, CH-NH); 4,00-3,87 (m, 2H, C=C-CHOH, $CH_2OH$); 3,77-3,67 (m, 1H, $CH_2OH$), 2,95-2,85 (m, 2H, OH); 2,28-2,18 (t, 2H, CO-$CH_2$); 2,11-2,00 (m, 2H, C=C-$CH_2$, J= 7,6 Hz); 1,75-1,58 (m, 2H, CO-$CH_2$); 1,45-1,11 (m, 54H, aliphat); 0,95-0,83 (t, 6H, -$CH_3$).

(ℓ) D,L-erythro-1,3-Dihydroxy-2-tetracosanoylamino-4-trans-eicosen

Durchführung der Synthese nach der allgemeinen Vorschrift. Zum Lösen des Tetracosanoylchlorids werden allerdings 100 ml wasserfreier Ether benötigt. Ausbeute: 2,64 g (64 %). Smp. 93-94°C; $R_F$= 0,15, Dichlormethan/Methanol 95:5.

Elementaranalyse für $C_{44}H_{87}NO_3$ (687,09):

| | C | H | N |
|---|---|---|---|
| berechnet: | C 77,93 | H 12,92 | N 2,06 |
| gefunden : | 77,98 | 13,02 | 2,21 |

$^1H$-NMR (in $CDCl_3$): 6,30-6,25 (d, 1H, N$\underline{H}$, J= 8,8 Hz); (m, 1H, C$\underline{H}$=C-CHOH); 5,59-5,47 (dd, 1H, CH=C$\underline{H}$-CHOH, $J_{trans}$= 15,5 Hz, $J_{vic}$= 6,7 Hz); 4,36-4,27 (m, 1H, C$\underline{H}$-NH); 4,00-3,87 (m, 2H, C=C-C$\underline{H}$OH, C$\underline{H}_2$OH); 3,75-3,65 (m, 1H, C$\underline{H}_2$OH); 2,85-2,73 (m, 2H, OH); 2,27-2,17 (t, 2H, CH-C$\underline{H}_2$, J= 7,6 Hz); 2,10-2,00 (m, 2H, C=C-C$\underline{H}_2$); 1,70-1,55 (m, 2H, CO-$CH_2$-C$\underline{H}_2$); 1,44-1,15 (m, 66H, aliphat); 0,93-0,82 (t, 6H, -C$\underline{H}_3$).

Beispiel 1

D- bzw. L-erythro-1-O-β-D-Glucopyranosyloxy-3-hydroxy-2-palmitoylamino-4-trans-octadecen

(1) D,L-erythro-3-Hydroxy-2-palmitoylamino-1-(triphenylmethyloxy)-4-trans-octadecen

Die Verbindung wird analog dem Verfahren gemäss Chem. Phys. Lipids 3, 59-69 (1969) hergestellt.

1,08 g (2 mMol) Verbindung (j) werden in einer Mischung aus 6 ml wasserfreiem Pyridin, 6 ml wasserfreiem Chloroform und 6 ml wasserfreiem Tetrahydrofuran gelöst und mit 0,56 g (4 mMol) Tritylchlorid versetzt. Nach einer Reaktionszeit von 48 Std. bei Raumtemperatur giesst man auf Wasser und extrahiert mit Ether. Nach Trocknen über Natriumsulfat und Einengen wird über Kieselgel mit Toluol/Essigester 9:1 chromatographiert.

Für die Analyse wird über Mitteldruck mit Toluol/Essigester 9:1 chromatographiert. Ausbeute: 0,94 g (60 %).

Smp. 58-60°C; $R_F$ = 0,64, Toluol/Aceton 8:2 (gelbbraune Färbung mit $H_2SO_4$) [Verbindung (e): $R_F$ = 0,12].

$^1$H-NMR (250 MHz, $CDCl_3$ in ppm):
7,3 (m, 15H, Trityl); 6,07 (d, 1H, NH, J = 7,6 Hz); 5,62 (td, 1H, $-CH_2-\underline{CH}=C$, J = 7,6 Hz); J = 15,2 Hz);, 5,25 (dd, 1H, C=$\underline{CH}$-CH, J = 6,1 Hz, J = 15,2 Hz); 4,18 (m, 1H, -CH-N); 4,05 (m, 1H, -CH-O); 3,34 (dd, 1H, $-CH_2-O$); 3,28 (dd, 1H, $-CH_2-O$, J = 4,0 Hz, J = 9,8 Hz); 2,2 (dd, 2H, $CO-CH_2-$, J = 7,9 Hz).

(2) D- bzw. L-erythro-3-[L(+)-O-Acetylmandeloyloxy]-2-palmitoylamino-1-(triphenylmethyloxy)-4-trans-octadecen

220 mg (0,28 mMol) der gemäss obigem Abschnitt erhaltenen Verbindung D-(1) bzw. L-(1) und 240 mg (0,84 mMol) L-(+)-Acetylmandelsäurechlorid werden in 5 ml wasserfreiem Toluol und 1,2 ml wasserfreiem Pyridin gelöst. Nach einer Stunde ist das Pyridinhydrochlorid vollständig ausgefallen. Man verdünnt mit 10 ml Ether und wäscht mit Wasser, trocknet über Natriumsulfat und engt ein. Die beiden Isomeren werden über Mitteldruckchromatographie mit Toluol/Essigester 95:5 getrennt. Ausbeute: 200 mg (75 % insgesamt; Verbindung D-(2): 38 %, Verbindung L-(2): 37 %).

Verbindung D-(2): $R_F$ = 0,44, Verbindung L-(2): $R_F$ = 0,51 [Verbindung (ℓ): $R_F$ = 0,25], Toluol/Essigester 9:1.

20.11.84

$^1$H-NMR (250 MHz, $CDCl_3$ in ppm): Verbindung D-(2): 7,35 (m, 15H, Trityl); 5,85 (d, 1H, NH, J = 9,8 Hz); 5,83 (s, 1H, -CH(Ph)OAc); 5,56 (m, 1H, -CH-O-Mandeloyl); 5,46 (td, 1H, $-CH_2-\underline{CH}=C$, J = 7,6 Hz, J = 15,2 Hz); 5,1 (dd, 1H, $C=\underline{CH}-CH$, J = 6,1 Hz, J = 15,2 Hz); 4,3 (m, 1H, -CH-N); 3,35 (dd, 1H, $-CH_2-O$, J = 9,4 Hz, J = 5 Hz); 3,19 (dd, 1H, $-CH_2-O$, J = 9,4 Hz, J = 5 Hz).

Verbindung L-(2): 7,3 (m, 15H, Trityl) 5,79 (s, 1H, -CH(Ph)OAc); 5,64 (td, 1H, $-Ch_2-CH=C$, J = 15,2 Hz, J = 7,6 Hz); 5,39 (m, 1Hz, -CH-O Mandeloyl); 5,25 (dd, 1H, $-C=\underline{CH}-CH$, J = 15,2 Hz, J = 6,9 Hz); 4,22 (m, 1H, -CH-N); 3,08 (dd, 1H, $-CH_2O$, J = 9,4 Hz, J = 5,0 Hz); 2,95 (dd, 1H, $-CH_2-O$, J = 9,4 Hz, J = 6,25 Hz).

(3) D- bzw. L-erythro-3-Hydroxy-2-palmitoylamino-1-(triphenylmethyloxy)-4-trans-octadecen

1 g (1,05 mMol) Verbindung D-(2) bzw. L-(2) wird in 50 ml wasserfreiem Methanol und 10 ml wasserfreiem Toluol gelöst und mit 0,1 ml 1M Natriummethanolatlösung versetzt und bei Raumtemperatur gerührt. Nach 12 Std. wird mit Ionenaustauscher neutralisiert, filtriert und eingeengt.
Ausbeute: 0,8 g (95 %).

Verbindung D-(3) bzw. L-(3): $R_F$ = 0,64, Toluol/Aceton 8:2. Die $^1$H-NMR-Daten der zwei Verbindungen sind mit jenen der Verbindung vom Abschnitt (1) identisch.

(4) D- bzw. L-erythro-3-Benzoyloxy-2-palmitoylamino-1-(triphenylmethyloxy)-4-trans-octadecen

930 mg (1,2 mMol) Verbindung D-(3) bzw. L-(3) und 0,6 ml (6 mMol) Benzoylchlorid werden in 20 ml wasserfreiem Toluol und 3 ml wasserfreiem Pyridin gelöst und 1 1/2 Std. bei Raumtemperatur gerührt. Man verdünnt mit 10 ml Ether, wäscht mit gesättigter Natriumhydrogencarbonatlösung, trocknet mit Natriumsulfat und engt ein. Kieselgelchromatographie mit Toluol/Essigester 9:1 liefert reines Produkt. Ausbeute: 1 g (95 %).

$R_F$ = 0,48, Toluol/Essigester 9:1. $^1$H-NMR (250 MHz, $CDCl_3$ in ppm): 7,93 (m, 2H, Benzoyl); 7,55 (m, 1H, Benzoyl); 7,4 (m, 8H, Benzoyl, Trityl); 5,88 (td, 1H, $-CH_2-\underline{CH}=C$, J = 15,2 Hz, J = 6,7 Hz); 5,7 (m, 2H, NH, -CH-OBz); 5,44 (dd, 1H, $C=\underline{CH}-CH$, J = 15,2 Hz, J = 7,6 Hz); 4,49 (m, 1H, CH-N); 3,45 (dd, 1H, $-CH_2-O$, J = 9,2 Hz, J = 3,4 Hz); 3,2 Hz (dd, 1H, $-CH_2-O-$, J=9,2 Hz, J = 4 Hz).

(5) D- bzw. L-erythro-3-Benzoyloxy-1-hydroxy-2-palmitoylamino-4-trans-octadecen

400 mg (0,45 mMol) Verbindung D-(4) bzw. L-(4) werden in 5 ml wasserfreiem Toluol gelöst und mit 0,18 ml wasserfreiem Methanol und 0,1 ml Bortrifluoridetherat versetzt. Nach 10 Min. war kein Ausgangsprodukt mehr festzustellen. Man verdünnt mit 5 ml Toluol, wäscht mit Wasser, trocknet über Natriumsulfat und engt ein. Zur Reinigung wird über Kieselgel mit Toluol/Aceton 9:1 chromatographiert. Ausbeute: 200 mg (70 %).

$R_F$ = 0,44, Toluol/Aceton 8:2. $^1$H-NMR (250 MHz, $CDCl_3$ in ppm): 8,04 (m, 2H, Benzoyl); 7,6 (m, 1H, Benzoyl); 7,47 (m, 2H, Benzoyl); 6,05 (d, 1H, NH, J = 8,8 Hz); 5,86 (td, 1H, $-CH_2-\underline{CH}=C$, J = 14,7 Hz, J = 6,7 Hz); 5,61 (dd, 1H, $-C=\underline{CH}-CH$, J = 14,7 Hz, J = 7,6 Hz); 5,53 (m, 1H, -CH-OBz); 4,29 (m, 1H, -CH-N); 3,72 (m, 2H, $-CH_2-O$); 2,9 (m, 1H, -OH).

(6) D- bzw. L-erythro-3-Benzoyloxy-2-palmitoylamino-1-(2,3,4,6-tetra-O-acetyl-β-D-glucopyranosyloxy)-4-trans-octadecen

100 mg (0,16 mMol) Verbindung D-(5) bzw. L-(5) und 180 mg (0,32 mMol) O-(2,3,4,6-Tetra-O-acetyl-α-D-glucopyranosyl)-trichloracetimidat werden in 10 ml wasserfreiem Methylenchlorid gelöst und mit einer Spatelspitze pulverisiertem Molekularsieb 0,4 nm (4 Å) und 2 ml 0,1 M Bortrifluorid-etherat in Methylenchlorid versetzt. Nach 3 Std. wird mit 10 ml Chloroform verdünnt, vom Molekularsieb filtriert, mit gesättigter Natriumhydrogencarbonatlösung gewaschen, über Natriumsulfat getrocknet und eingeengt. Zur Reinigung wird über Kieselgel mit Toluol/Aceton 9:1 filtriert und über Mitteldruck mit Toluol/Aceton 9:1 chromatographiert. Ausbeute: 78 mg (50 %). $R_F$ = 0,55, Toluol/Aceton 8:2.

$^1$H-NMR (250 MHz, $CDCl_3$ in ppm):
Verbindung D-(6): 8,0 (m, 2H, Benzoyl); 7,57 (m, 1H, Benzoyl); 7,44 (m, 2H, Benzoyl); 5,81 (m, 2H, NH, $CH_2-\underline{CH}=C$); 5,42 (m, 2H, $C=\underline{CH}-\underline{CH}-OBz$); 5,15 (dd, 1H, H-4, J = 7,5 Hz, J = 7,5 Hz); 5,01 (m, 2H, H-3, H-2); 4,47 (m, 1H, NH); 3,39 (d, 1H, H-1, J = 7,9 Hz), 4,23 (dd, 1H, H-6, J = 12,2 Hz, J = 4,9 Hz); 4,04 (dd, 1H, H-6', J = 12,2 Hz, J = 2,1 Hz); 3,9 (dd, 1H, $-CH_2-O-$, J = 9,8 Hz, J = 3,05 Hz); 3,68 (m, 2H, $-CH_2-O$, H-5); 2,1 (s, 3H, Acetyl); 2,04 (s, 3H, Acetyl); 1,99 (s, 6H, Acetyl).

Verbindung L-(6): 8,04 (m, 2H, Benzoyl); 7,58 (m, 1H, Benzoyl); 7,45 (m, 2H, Benzoyl); 5,95-5,72 (m, 2H, NH, $-CH_2-\underline{CH}=C$); 5,6-5,3 (m, 2, $-C=\underline{CH}-OBz$); 5,25-4,95 (m, 3H, H-4, H-3, H-2); 4,45 (m, 2H, $-H-1-\underline{CH}-N$); 4,3-3,85 (m, 3H); 3,65 (m, 2H).

(7) D- bzw. L-erythro-1-(β-D-Glucopyranosyloxy)-3-hydroxy-2-palmitoylamino-4-trans-octadecen

100 mg (0,1 mMol) Verbindung D-(6) bzw. L-(6) werden in 5 ml wasserfreiem Methanol suspendiert und mit einer katalytischen Menge Natrium-Metall versetzt. Nach 15 Min. wird mit Ionenaustauscher der sauren Form neutralisiert; dabei trübt sich die Lösung. Man erwärmt und filtriert. Nach Einengen wird zur Reinigung über eine kurze Kieselgelsäule mit Chloroform/Methanol 9:1 chromatographiert. Ausbeute: 70 mg (100 %). $R_F$ = 0,4, Chloroform/Methanol 85:15.

$^1$H-NMR (250 MHz, DMSO in ppm):
Verbindung D-(7): 7,5 (d, 1H, NH, J = 8,7 Hz); 5,52 (m, 1H, $-CH_2-\underline{CH}=C$); 5,35 (dd, 1H, C=CH-, J = 15,2 Hz, J = 6,5 Hz); 5,03 (d, 1H, OH, J = 4,3 Hz); 4,92 (m, 3H, OH); 4,5 (t, 1H, OH, J = 4,9 Hz); 4,09 (d, 1H, H-1, J = 8,2 Hz); 4,0-3,55 (m, 4H) 3,45 (m, 2H); 3,15-2,9 (m, 4H); 2,1-1,9 (m, 4H); 1,45 (m, 2H); 1,22 (brs, 46H, $-CH_2-$); 0,85 (m, 6H, $CH_3$).

Verbindung L-(7): 7,47 (d. 1H. NH, J = 9,1 Hz); 5,52 (td, 1H, $CH_2-\underline{CH}=C$, J = 15,2 Hz, J = 6,1 Hz); 5,34 (dd, 1H, $C=\underline{CH}-CH$, J = 15,2 Hz, J = 6,7 Hz); 4,9 (m, 3H, OH); 4,58 (m, 1H, OH); 4,13 (d, 1H, H-1, J = 7,3 Hz); 4,0-2,91 (m, 10H); 2,12-1,85 (m, 4H, $CO-\underline{CH_2}$, $C=C-\underline{CH_2}$); 1,58-1,09 (m, 48H, $CH_2$ aliphat.); 0,85 (m, 6H, $CH_3$).

Beispiel 2

(8) D,L-erythro-3-Hydroxy-1-(diphenyl-p-methoxyphenylmethyloxy)-2-stearoylamino-4-trans-eicosen

2 g (3,3 mMol) Verbindung (k) und 1,56 g (5 mMol) Monomethoxytritylchlorid werden in 30 ml eines Gemisches aus jeweils wasserfreiem Tetrahydrofuran, Chloroform und Pyridin 1:1:1 2 Std. lang bei Raumtemperatur gerührt. Man giesst auf 100 ml Wasser und extrahiert zweimal mit 50 ml Ether. Die organische Phase wird über Natriumsulfat getrocknet und bis zur Trockene eingeengt. Man chromatographiert über Kieselgel mit Toluol/Essigester 8,5:1,5. Ausbeute: 2,1 g (73 %). Smp. 49-51°C. $R_F$ = 0,27, Toluol/Essigester 8,5:1,5.

Elementaranalyse für $C_{58}H_{91}NO_4$ (866,28):

| | | | |
|---|---|---|---|
| berechnet: | C 80,42 | H 10,58 | N 1,62 |
| gefunden : | 80,23 | 10,79 | 1,74 |

$^1$H-NMR (in $CDCl_3$): 7,42-7,20 (m, 12 H, aromat.); 6,87-6,80 (m, 2H, aromat.); 6,10-6,04 (d, 1H, NH, J = 7,9 Hz); 5,73-5,57 (m, 1H, C$\underline{H}$=CH-CHOH); 5,32-5,20 (dd, 1H, CH=C$\underline{H}$-CHOH, $J_{vic}$ = 6,4 Hz, $J_{trans}$ = 15,5 Hz); 4,22-4,14 (m, 1H, C$\underline{H}$-NH); 4,10-4,02 (m, 1H, C$\underline{H}$-OH); 3,79 (s, 3H, $OCH_3$); 3,45-3,28 (m, 3H, OH, C$\underline{H}_2$OH); 2,25-2,17 (t, 2H, C=C-C$\underline{H}_2$, J = 7,6 Hz); 1,97-1,88 (m, 2H, C$\underline{H}_2$-C=C); 1,70-1,60 (m, 2H, C=C-$CH_2$-C$\underline{H}_2$); 1,40-1,15 (m, 54H, aliphat.); 0,95-0,82 (t, 6H, $-CH_3$).

(9) D- bzw. L-erythro-3-[L(+)-O-Acetylmandeloyloxy]-1-(diphenyl-p-methoxyphenylmethyloxy)-2-stearoylamino-4-trans-eicosen

4,3 g (5 mMol) Verbindung D,L-(8) werden in 50 ml eines Gemisches aus wasserfreiem Toluol und wasserfreiem Pyridin 4:1 gelöst. 3,2 g (15 mMol) L(+)-O-Acetylmandelsäurechlorid werden hinzugegeben. Man lässt 2 Std. bei Raumtemperatur rühren. Nach Verdünnen mit 100 ml Ether wird zweimal mit 50 ml Wasser ausgeschüttelt. Die organische Phase wird über Natriumsulfat getrocknet und eingeengt. Nach Chromatographieren über Kieselgel mit Toluol/Essigester 95:5 können die Diastereomeren D-(9) und L-(9) durch Mitteldruckchromatographie an Kieselgel mit Toluol/Essigester 96:4 getrennt werden.

Verbindung D-(9): Ausbeute: 1,8 g (35 %, bezogen auf die Gesamtmenge an Verbindung (k)). Smp. 59-61°C, $R_F$ = 0,44, Toluol/Essigester 95:5.

$^1$H-NMR (in $CDCl_3$): 7,50-7,17 (m, 17H, aromat.); 6,86-6,80 (m, 2H, aromat.); 5,85-5,80 (m, 2H, NH; CO-C<u>H</u>-OAc); 5,60-5,52 (m, 1H, C<u>H</u>-OAc.mand.); 5,45-5,30 (m, 1H, C<u>H</u>=CH-CHO-); 5,17-5,05 (dd, 1H, CH=C<u>H</u>-CHO-, $J_{trans}$= 15,5 Hz, $J_{vic}$= 6,1 Hz); 4,35-4,24 (m, 1H, C<u>H</u>-NH-); 3,8 (s, 3H, $OCH_3$); 3,39-3,30 (dd, 1H, $C\underline{H}_2O$-, $J_{gem}$= 9,7 Hz, $J_{vic}$= 3,9 Hz); 3,22-3,14 (dd, 1H, $C\underline{H}_2O$-, $J_{gem}$= 9,7 Hz, $J_{vic}$= 4,2 Hz); 2,22 (s, 3H, OAc); 2,12-2,03 (t, 2H, CO-$C\underline{H}_2$, J= 7,6 Hz); 1,85-1,75 (m, 2H, C=C-$C\underline{H}_2$); 1,60-1,50 (m, 2H, CO-$CH_2$-$C\underline{H}_2$); 1,40-1,07 (m, 54H, aliphat.); 0,94-0,82 (t, 6H, $CH_3$).

Verbindung L-(9): Ausbeute: 1,6 g (31 %, bezogen auf die Gesamtmenge an Verbindung (k)). Smp. 34-35°C. $R_F$ = 0,52, Toluol/Essigester 95:5.

$^1$H-NMR (in $CDCl_3$): 7,50-7,17 (m, 17H, aromat.); 6,86-6,80 (m, 2H, aromat.); 5,88 (s, 1H, Co-C$\underline{H}$-OAc); 5,79-5,65 (m, 1H, C$\underline{H}$=CH-CHO-); 5,50-5,24 (m, 2H, CH=C$\underline{H}$-CHO-, C$\underline{H}$-OAc.mand.); 5,17-5,09 (d, 1H, NH, J=9,2 Hz); 4,35-4,24 (m, 1H, ·C$\underline{H}$NH); 3,80 (s, 3H, $OCH_3$); 3,18-3,10 (dd, 1H, $C\underline{H}_2$-O-, $J_{gem}$=9,7 Hz, $J_{vic}$=3,9 Hz); 3,06-2,98 (dd, 1H, $C\underline{H}_2$-O-, $J_{gem}$=9,7 Hz, $J_{vic}$=4,2 Hz); 2,10 (s, 3H, OAc); 1,89-1,80 (t, 2H, CO-$C\underline{H}_2$); 1,65-1,40 (m, 4H, C=C-$C\underline{H}_2$, CO-$CH_2$-$C\underline{H}_2$); 1,38-1,10 (m, 54H, aliphat.); 0,94-0,82 (t, 6H, $CH_3$).

Die Verbindungen D-(9) und L-(9) wurden durch Abspalten des Monomethoxytrityl- und Acetylmandeloylrestes in die entsprechenden enantiomeren Ceramide überführt. Die so erhaltenen Ceramide waren $^1$H-NMR-spektroskopisch mit der Verbindung (k) identisch.

(10) D-erythro-3-[L(+)-O-Acetylmandeloyloxy)-1-hydroxy-2-stearoylamino-4-trans-eicosen

1 g (0,95 mMol) Verbindung D-(9) wird in 50 ml eines Gemisches aus Dichlormethan und Methanol 4:1, welches 1% p-Toluolsulfonsäure enthält, gelöst. Man lässt eine Stunde bei Raumtemperatur rühren. Dann wird zweimal mit wässriger Natriumhydrogencarbonatlösung ausgeschüttelt. Nach Einengen wird über Kieselgel mit

Toluol/Essigester 7,5:2,5 chromatographiert. Ausbeute: 0,47 g (65 %). Smp. 76-77°C; $R_F$ = 0,46, Dichlormethan/Methanol 95:5.

$^1$H-NMR (in $CDCl_3$): 7,50-7,36 (m, 5H, aromat.); 6,06-6,00 (d, 1H, NH, J = 8,2 Hz); 5,78 (s, 1H, CO-C$\underline{H}$-OAc); 5,57-5,28 (m, 2H, C$\underline{H}$=C$\underline{H}$); 4,25-4,13 (m, 1H, C$\underline{H}$-NH); 3,87-3,75 (m, 1H, C$\underline{H}_2$OH); 3,72-3,60 (m, 1H, C$\underline{H}_2$OH); 2,52-2,40 (m, 1H, OH); 2,23 (s, 3H, OAc) 2,22-2,13 (t, 2H, CO-C$\underline{H}_2$, J = 7,6 Hz); 1,95-1,83 (m, 2H, C=C-C$\underline{H}_2$); 1,65-1,52 (m, 2H, CO-$CH_2$-C$\underline{H}_2$); 1,37-1,10 (m, 54H, aliphat.); 0,95-0,82 (t, 6H, $CH_3$)

## Beispiel 3

(12) D,L-erythro-3-Hydroxy-2-stearoylamino-1-(triphenylmethyloxy)-4-trans-eicosen

2,4 g (4 mMol) Verbindung (k) und 2,5 g (8,9 mMol) Tritylchlorid werden in 45 ml eines Gemisches aus jeweils wasserfreiem Tetrahydrofuran, Chloroform und Pyridin 1:1:1 48 Std. lang bei Raumtemperatur gerührt. Man giesst die Lösung auf 200 ml Wasser und extrahiert zweimal mit 100 ml Ether. Die Etherphase wird zweimal mit 50 ml 0,1N Salzsäure gewaschen und anschliessend mit 100 ml wässriger Natriumhydrogencarbonatlösung ausgeschüttelt. Man trocknet über Natriumsulfat und engt bis zur Trockene ein. Danach wird über Kieselgel mit Toluol/Essigester 9:1 chromatographiert. Ausbeute: 2 g (60 %); Smp. 69-70°C; $R_F$ = 0,55, Toluol/Aceton 8:2.

Elementaranalyse für $C_{57}H_{89}NO_3$ (836,26):

| | C | H | N |
|---|---|---|---|
| berechnet: | C 81,86 | H 10,71 | N 1,67 |
| gefunden : | 81,74 | 10,72 | 1,74 |

$^1H$-NMR (in $CDCl_3$): 7,44-7,20 (m, 15H, aromat.); 6,11-6,05 (d, 1H, NH, J=7,6 Hz); 5,71-5,57 (m, 1H, C<u>H</u>=CH-CHOH); 5,32-5,20 (dd, 1H, CH=C<u>H</u>-CHOH, $J_{trans}$ =15,5 Hz, $J_{vic}$=5,7 Hz); 4,23-4,14 (m, 1H, C<u>H</u>-NH); 4,12-4,02 (m, 1H, -C<u>H</u>OH); 3,45-3,36 (m, 2H, O<u>H</u>, C<u>H</u>$_2$OH); 3,34-3,27 (dd, 1H, C<u>H</u>$_2$OH, $J_{gem}$=9,5 Hz, $J_{vic}$=3,6 Hz); 2,27-2,18 (t, 2H, CO-C<u>H</u>$_2$, J=7,6 Hz); 1,98-1,88 (m, 2H, C=C-C<u>H</u>$_2$); 1,71-1,60 (m, 2H, CH-$CH_2$-C<u>H</u>$_2$); 1,40-1,14 (m, 54H, aliphat,); 0,95-0,83 (t, 6H, -$CH_3$).

Beispiel 4

D- bzw. L-erythro-3-Hydroxy-1-(β-D-glucopyranosyloxy)-2-palmitoylamino-4-trans-octadecen

(13) D- bzw. L-erythro-3-[L(+)-O-Acetylmandeloyloxy]-1-hydroxy-2-palmitoylamino-4-trans-octadecen

400 mg (0,42 mMol) Verbindung D-(2) bzw. L-(2) aus Beispiel 1(2) werden in 4 ml wasserfreiem Toluol gelöst und mit 0,2 ml wasserfreiem Methanol versetzt. Unter Feuchtigkeitsausschluss und starkem Rühren werden 0,1 ml (0,84 mMol) Bortrifluorid-etherat zugegeben, wobei sich die Lösung kurzzeitig gelb anfärbt. Nach 15 Min. war kein Ausgangsprodukt mehr vorhanden (DC). Man verdünnt mit Toluol, wäscht mit Wasser, trocknet über Natriumsulfat und entfernt das Lösungsmittel im Vakuum.

Zur Reinigung wird über Kieselgel mit Toluol/Aceton 9:1 chromatographiert. Ausbeute: 180 mg (60 %).

Verbindung D-(13): $R_F$ = 0,4, Toluol/Aceton 8:2;
Verbindung L-(13): $R_F$ = 0,44, Toluol/Aceton 8:2.

$^1$H-NMR (250 MHz, $CDCl_3$ in ppm):
Verbindung D-(13): 7,41 (m, 5H, Phenyl); 6,2 (d, 1H, NH, J = 8,2 Hz); 5,8 (s, 1H, -<u>CH</u>-OAc); 5,6-5,25 (m, 3H, CH=CH, -CH-O-Mandeloyl); 4,2 (m, 1H, CH-N); 3,81 (dd, 1H, -<u>$CH_2$</u>-OH, J = 12,7 Hz, J = 4,8 Hz); 3,65 (dd, 1H, -<u>$CH_2$</u>-OH, J = 12,7 Hz); 2,45 (m, 1H, OH); 2,2 (s, 3H, -CO-$CH_3$); 2,18 (m, 2H, aliphat.); 1,9 (m, 2H, aliphat.); 1,55 (m, 4H, aliphat.); 1,22 (m, 44H, aliphat.); 0,88 (m, 6H, -$CH_3$).

Verbindung L-(13): 7,4 (m, 5H, Phenyl); 5,86 (s, 1H, -<u>CH</u>-OAc); 5,8-5,62 (m, 2H, -<u>CH</u>=CH-CH-O, NH); 5,48-5,3 (m, 2H, -CH=<u>CH</u>-<u>CH</u>-O-Mandeloyl); 4,0 (m, 2H, -CH-N, OH); 3,5 (dd, 1H, -$CH_2$-O, J = 4,6 Hz); 3,35 (dd, 1H, -$CH_2$-O, J = 11,6 Hz, J = 5,2 Hz); 2,2 (s, 3H, -CO-$CH_3$); 1,96 (m, 4H, aliphat.); 1,5 (m, 2H, aliphat.); 1,2 (m, 46H, aliphat.); 0,85 (m, 6H, -$CH_3$).

Elementaranalyse für $C_{44}H_{75}NO_6$ (714,09):

| | | | |
|---|---|---|---|
| berechnet: | C 74,01 | H 10,59 | N 1,96 |
| gefunden : | 73,48 | 10,64 | 2,06 |

(14) <u>D- bzw. L-erythro-1-(2,3,4,6-Tetra-O-acetyl-ß-D-glucopyranosyloxy)-3-[L(+)-O-acetylmandeloyloxy]-2-palmitoylamino-4-trans-octadecen</u>

350 mg (0,49 mMol) Verbindung D-(13) bzw. L-(13) und 400 mg (0,8 mMol) O-(2,3,4,6-Tetra-O-acetyl-α-D-gluco-

pyranosyl)-trichloracetimidat werden in 30 ml wasserfreiem Methylenchlorid gelöst und mit einer Spatelspitze pulverisiertem Molekularsieb 0,4 nm (4 Å) versetzt. Unter Feuchtigkeitsausschluss und gutem Rühren wird 1 ml Bortrifluorid-etherat zugegeben. Nach 2 Std. ist kein Ausgangsprodukt mehr vorhanden (DC). Man wäscht mit gesättigter Natriumhydrogencarbonatlösung, trocknet über Natriumsulfat und entfernt das Lösungsmittel im Vakuum. Zur Reinigung wird über Kieselgel mit Toluol/Aceton 9:1 und anschliessend noch über Mitteldruck mit Toluol/Aceton 9:1 chromatographiert. Ausbeute: 300 mg (59 %).

Verbindung D-(14): $R_F$ = 0,58, Toluol/Aceton 8:2;
Verbindung L-(14): $R_F$ = 0,6, Toluol/Aceton 8:2.

Elementaranalyse für $C_{58}H_{93}NO_{15}$ (1 044,37):

| | | | |
|---|---|---|---|
| berechnet: | C 66,70 | H 8,98 | N 1,34 |
| gefunden : | 66,16 | 9,11 | 1,24 |

(15) D- bzw. L-erythro-3-Hydroxy-1-(β-D-glucopyranosyloxy)-2-palmitoylamino-4-trans-octadecen

120 mg (0,11 mMol) Verbindung D-(14) bzw. L-(14) werden in 6 ml wasserfreiem Methanol gelöst, mit 0,05 ml einer 1 M Natriummethanolatlösung versetzt und bei Raumtemperatur gerührt. Nach 2 Std. neutralisiert man mit Ionenaustauscher der sauren Form, wobei eine leichte Trübung eintritt. Man erwärmt, filtriert vom Ionenaustauscher ab, wäscht mit Methanol nach und dampft bis zur Trockne ein. Zur Reinigung wird über Kieselgel mit Chloroform/Methanol 85:15 chromatographiert. Ausbeute: 75 mg (97 %).

Verbindung D-(15): $R_F$ = 0,6, Chloroform/Methanol 8:2;

Verbindung L-(15): $R_F$ = 0,6, Chloroform/Methanol 8:2.

Die Verbindungen sind mit den nach Beispiel 1 erhaltenen Verbindungen D-(7) bzw. L-(7) identisch.

Beispiel 5

D- bzw. L-erythro-1-O-ß-D-Glucopyranosyloxy-3-hydroxy-2-palmitoylamino-4-trans-octadecen

(16) D,L-erythro-3-Benzoyloxy-2-palmitoylamino-1-(triphenylmethyloxy)-4-trans-octadecen

250 mg (0,32 mMol) D,L-erythro-3-Hydroxy-2-palmitoylamino-1-(triphenylmethyloxy)-4-trans-octadecen [Verbindung (1)] werden in 6 ml wasserfreiem Toluol und 1 ml wasserfreiem Pyridin gelöst. Dazu gibt man 0,25 ml (1,5 mMol) Benzoylchlorid und lässt 1 1/2 Std. bei Raumtemperatur rühren. Man verdünnt mit 10 ml Ether, wäscht mit gesättigter Natriumhydrogencarbonatlösung, trocknet über Natriumsulfat und engt bis zur Trockne ein. Zur Reinigung wird über Kieselgel mit Toluol/Essigester 9:1 chromatographiert. Das Produkt ist mit der nach Beispiel 1 dargestellten Verbindung D-(4) bzw. L-(4) $^1$H-NMR-spektroskopisch identisch. Ausbeute: 260 mg (92 %). $R_F$ = 0,48, Toluol/Essigester 9:1.

(17) D,L-erythro-3-Benzoyloxy-1-hydroxy-2-palmitoylamino-4-trans-octadecen

700 mg (0,79 mMol) D,L-erythro-3,O-Benzoyl-2-palmitoylamino-1-(triphenylmethyloxy)-4-trans-octadecen [Verbindung (16)] werden in 10 ml wasserfreiem Toluol gelöst und mit 0,25 ml wasserfreiem Methanol und 0,14 ml Bortrifluorid-etherat versetzt. Nach 10 Min. war kein

Ausgangsprodukt mehr vorhanden (DC). Man verdünnt mit 10 ml Toluol, wäscht mit Wasser, trocknet über Natriumsulfat und engt bis zur Trockne ein. Zur Reinigung wird über Kieselgel mit Toluol/Aceton 9:1 chromatorgraphiert. Das Produkt ist mit der nach Beispiel 1 dargestellten Verbindung D-(5) bzw. L-(5) $^1$H-NMR-spektroskopisch identisch. Ausbeute: 350 mg (69 %). $R_F$ = 0,44, Toluol/Aceton 8:2.

(18) D- bzw. L-erythro-3-Benzoyloxy-2-palmitoylamino-1-(2,3,4,6-tetra-O-acetyl-β-D-glucopyranosyloxy)-4-trans-octadecen

200 mg (0,31 mMol) D,L-erythro-3-Benzoyloxy-1-hydroxy-2-palmitoylamino-4-trans-octadecen [Verbindung (17)] und 200 mg (0,4 mMol) O-(2,3,4,6-Tetra-O-acetyl-α-D-glucopyranosyl)-trichloracetimidat werden in 10 ml wasserfreiem Methylenchlorid gelöst und mit einer Spatelspitze pulverisiertem Molekularsieb 0,4 nm (4 Å) versetzt. Nach Zugabe von 0,4 ml einer 0,1 M Trimethylsilyltrifluoracetatlösung in Methylenchlorid lässt man noch 6 Std. bei Raumtemperatur rühren. Man verdünnt mit Chloroform, filtriert vom Molekularsieb ab, wäscht mit gesättigter Natriumhydrogencarbonatlösung, trocknet über Natriumsulfat und engt bis zur Trockne ein. Man chromatographiert über Kieselgel mit Toluol/Aceton 9:1 und zur Trennung der Diastereomeren wird unter Mitteldruck mit Toluol/Aceton 9:1 an Kieselgel chromatographiert. Die erhaltenen Produkte sind mit den nach Beispiel 1 dargestellten Verbindungen D-(6) bzw. L-(6) identisch. Ausbeute: 160 mg (50 %, 25 % D- und 25 % L-Verbindung).

20.11.84

Verbindung D-(18): $R_F$ = 0,55, Toluol/Aceton 8:2;
Verbindung L-(18): $R_F$ = 0,54, Toluol/Aceton 8:2.

Die Abspaltung der Acetylgruppen wird nach der in Beispiel 1(7) beschriebenen Methode durchgeführt; die erhaltenen Produkte erweisen sich mit den nach Beispiel 1 hergestellten Verbindungen D-(7) bzw. L-(7) identisch.

Beispiel 6

D- bzw. L-erythro-1-O-ß-D-Glucopyranosyloxy-3-hydroxy-2-tetracosanoylamino-4-trans-eicosen

(19) D,L-erythro-3-Hydroxy-1-(diphenyl-p-methoxyphenyl-methyloxy)-2-tetracosanoylamino-4-trans-eicosen

6 g (7,2 mMol) Verbindung (ℓ) und 3,43 g (11 mMol) Monomethoxytritylchlorid werden in 50 ml wasserfreiem Pyridin 5 Stunden lang bei Raumtemperatur gerührt. Man giesst auf 200 ml Wasser und extrahiert zweimal mit 100 ml Ether. Die organische Phase wird über Natriumsulfat getrocknet und bis zur Trockne eingeengt. Man chromatographiert über Kieselgel mit Toluol/Essigester 8,5:1,5. Ausbeute: 4,13 g (65 %), $R_F$ = 0,27, Toluol/Essigester 85:15.

(20) D,L-erythro-3-Benzoyloxy-1-(diphenyl-p-methoxyphenyl-methyloxy)-2-tetracosanoylamino-4-trans-eicosen

4 g (4,2 mMol) Verbindung (19) und 5 g (34 mMol) Benzoylchlorid werden in 30 ml wasserfreiem Pyridin 12 Stunden lang bei Raumtemperatur gerührt. Man giesst auf 200 ml Wasser und extrahiert zweimal mit 100 ml Ether. Die organische Phase wird über Natriumsulfat getrocknet und eingedampft. Man chromatographiert über Kieselgel mit Toluol/Essigester 9:1. Ausbeute: 3 g (66 %). $R_F$ = 0,64, Toluol/Essigester 85:15.

(21) D,L-erythro-3-Benzoyloxy-1-hydroxy-2-tetracosanoyl-amino-4-trans-eicosen

3 g (2,8 mMol) Verbindung (20) werden eine Stunde lang bei Raumtemperatur in 50 ml eines Gemisches aus Dichlormethan/Methanol 4:1, das 1 Gewichtsprozent p-Toluolsulfonsäure enthält, gerührt. Man schüttelt mit 30 ml gesättigter, wässriger Natriumhydrogencarbonatlösung aus und dampft die organische Phase, nach Trocknen über Natriumsulfat, ein. Es wird über Kieselgel mit Dichlormethan/Methanol 97:3 chromatographiert. Ausbeute: 1,5 g (67 %). $R_F$ = 0,58 Dichlormethan/Methanol 95:5.

(22) D- bzw. L-erythro-3-Benzoyloxy-2-tetracosanoylamino-1-(2,3,4,6-tetra-O-acetyl-ß-D-glucopyranosyloxy)-4-trans-eicosen

150 mg (0,19 mMol) Verbindung (21) und 180 mg (0,32 mMol) O-(2,3,4,6-Tetra-O-acetyl-α-D-glucopyranosyl)-trichloracetimidat werden in 10 ml wasserfreiem Methylenchlorid gelöst und mit einer Spatelspitze pulverisiertem Molekularsieb 0,4 nm (4 Å) und 2 ml 0,1 M Bortrifluoridetherat in Methylenchlorid versetzt. Nach 3 Stunden wird mit 10 ml Chloroform verdünnt, vom Molekularsieb filtriert, mit gesättigter Natriumhydrogencarbonatlösung gewaschen, über Natriumsulfat getrocknet und eingeengt. Zur Reinigung wird über Kieselgel mit Toluol/Aceton 9:1 filtriert.

Die Diastereomeren D-(22) bzw. L-(22) werden durch Chromatographieren über Mitteldruck mit Toluol/Aceton 9:1 getrennt.

Ausbeute:

D-(22) 40 mg (18 %); $R_F$ = 0,55 Toluol/Aceton 8:2

L-(22) 40 mg (18 %); $R_F$ = 0,52 Toluol/Aceton 8:2

$^1$H-NMR (250 MHz, $CDCl_3$ in ppm):

Verbindung D-(22): 8,0 (m, 2H, Benzoyl); 7,57 (m, 1H, Benzoyl); 7,44 (m, 2H, Benzoyl); 5,81 (m, 2H, NH, $CH_2$-CH=C); 5,42 (m, 2H, C=CH-CH-OBz); 5,15 (dd, 1H, H-4, J=7,5 Hz , J=7,5 Hz); 5,01 (m, 2H, H-3, H-2); 4,47 (m, 1H, NH); 3,39 (d, 1H, H-1, J=7,9 Hz); 4,23 (dd, 1H, H-6, J=12,2 Hz, J=4,9 Hz); 4,04 (dd, 1H, H-6', J=12,2 Hz, J=2,1 Hz); 3,9 (dd, 1H, $-CH_2$-O-, J=9,8 Hz, J=3,05 Hz); 3,68 (m, 2H, $-CH_2$-O, H-5); 2,1 (s, 3H, Acetyl); 2,04 (s, 3H, Acetyl); 1,99 (s, 6H, Acetyl).

Verbindung L-(22): 8,04 (m, 2H, Benzoyl); 7,58 (m, 1H, Benzoyl); 7,45 (m, 2H, Benzoyl); 5,95-5,72 (m, 2H, NH, $-CH_2$-CH=C); 5,6-5,3 (m, 2H, -C=CH-OBz); 5,25-4,95 (m, 3H, H-4, H-3, H-2); 4,45 (m, 2H, H-1 CH-N); 4,3-3,85 (m, 3H); 3,65 (m, 2H).

(23) D- bzw. L-erythro-1-O-ß-D-Glucopyranosyloxy-3-hydroxy-2-tetracosanoylamino-4-trans-eicosen

55,6 mg (0,05 mMol) Verbindung D-(22) bzw. L-(22) werden in 3 ml wasserfreiem Methanol gelöst, mit 0,03 ml einer 1 M Natriummethanolatlösung versetzt und bei Raumtemperatur gerührt. Nach 1 Stunde neutralisiert man mit Ionenaustauscher der sauren Form, wobei eine leichte Trübung eintritt. Man erwärmt, filtriert vom Ionenaustauscher ab, wäscht mit Methanol nach und dampft zur Trockne ein. Zur Reinigung wird über Kieselgel mit Chlorofom-Methanol 85:15 chromatographiert. Ausbeute: 42 mg (95 %).

Verbindung D-(23): $R_F$ = 0,7, Chloroform/Methanol 8:2;
Verbindung L-(23): $R_F$ = 0,7, Chloroform/Methanol 8:2.

## Patentansprüche

1. Wundheilungsfördernde bzw. zell- und geweberegenerierende Sphingosinderivate der Formel (I)-D und (I)-L, in welchen $R^1$ den Acylrest einer Fettsäure mit 14 bis 24 Kohlenstoffatomen oder die entsprechenden Acylreste mit einer Hydroxylgruppe in α-Stellung oder mit einer oder zwei Doppelbindungen in cis-Konfiguration und $R^2$ den Pentadecanyl- oder Heptadecanylrest oder die entsprechenden $C_{15}$- und $C_{17}$-Reste mit einer, zwei oder drei Doppelbindungen, von welchen jeweils eine in 1,2-Stellung sitzt und trans-Konfiguration aufweist, die andere oder anderen, wenn vorhanden, cis-Konfiguration aufweisen, bedeutet.

2. Sphingosinderivate der Formel (I)-D nach Anspruch 1.

3. Sphingosinderivate der Formel (I)-D und (I)-L nach Anspruch 1, in welchen $R^1$ den Acylrest einer Fettsäure mit 14 bis 20 Kohlenstoffatomen oder die entsprechenden Acylreste mit einer Hydroxylgruppe in α-Stellung oder mit einer oder zwei Doppelbindungen in cis-Konfiguration bedeutet und $R^2$ die oben angegebene Bedeutung hat.

4. Sphingosinderivate der Formel (I)-D nach Anspruch 1, in welcher $R^1$ den Acylrest einer Fettsäure mit 14 bis 20 Kohlenstoffatomen oder die entsprechenden Acylreste mit einer Hydroxylgruppe in α-Stellung oder mit einer oder zwei Doppelbindungen in cis-Konfiguration bedeutet und $R^2$ die oben angegebene Bedeutung hat.

5. Als Sphingosinderivat nach Anspruch 1 das D- und das L-erythro-1-(β-D-Glucopyranosyloxy)-3-hydroxy-2-palmitoylamino-4-trans-octadecen.

6. Als Sphingosinderivat nach Anspruch 1 das D- und das L-erythro-1-(β-D-Glucopyranosyloxy)-3-hydroxy-2-tetracosanoylamino-4-trans-eicosen.

7. Pharmazeutische Zubereitung zur Behandlung von Wunden, wie Ulcus cruris, Geschwüre des Magendarmtraktes, insbesondere Ulcus ventriculi und Ulcus duodeni, diabetische Gängrän, Strahlenschäden, Verbrennungen und Hauttransplantationen, dadurch gekennzeichnet, dass der Wirkstoff ein Sphingosinderivat der Formel (I)-D oder (I)-L nach Anspruch 1 ist.

8. Verfahren zur Herstellung der Sphingosinderivate der Formel (I)-D oder (I)-L nach Anspruch 1, dadurch gekennzeichnet, dass man eine optisch aktive Verbindung der Formel (II)-D oder (II)-L, in welchen $R^1$ und $R^2$ die oben angegebenen Bedeutungen haben, oder das entsprechende Racemat mit einem organischen Reagens, das mit einer primären Hydroxylgruppe selektiv zu reagieren vermag, unter Bildung von Verbindungen der Formel (III), in welchen R eine Hydroxylschutzgruppe bedeutet, umsetzt,

(A) die Verbindung der Formel (III) mit einer organischen Carbonsäure unter Bildung einer Verbindung der Formel (IV), in welcher $Ac^1$ den Acylrest einer organischen Carbonsäure bedeutet, verestert oder

(B) bei Verwendung eines Racemates als Ausgangsprodukt die Verbindung der Formel (III) mit einer optisch aktiven organischen Säure verestert und das erhaltene Gemisch von diastereomeren Verbindungen der Formel (V), in welchen $Ac^2$ den Acylrest einer optisch aktiven organischen Säure bedeutet, in die Diastereomeren trennt oder

(C) auf die Variante (B) anschliessend, die einzelnen Diastereomeren der Formel (V) desacyliert und mit einer organischen Carbonsäure unter Bildung von enantiomeren Verbindungen der Formel (VI), in welchen

$Ac^3$ den Acylrest einer organischen Carbonsäure bedeutet, verestert, die bei der Variante (A) erhaltene Verbindung der Formel (IV) bzw. die bei der Variante (B) erhaltenen Diastereomeren der Formel (V) bzw. die bei der Variante (C) erhaltenen Enantiomeren der Formel (VI) von der Hydroxylschutzgruppe R unter Bildung entsprechender Verbindungen der Formel (VII), (VIII) bzw. (IX) befreit, die Verbindung der Formel (VII), (VIII) bzw. (IX) mit dem O-Trifluor- oder O-Trichloracetimidat einer D-Glucose, deren Hydroxylgruppen in den 2-, 3-, 4- und 6-Stellungen durch Acylreste $Ac^4$ geschützt sind, unter Bildung von Verbindungen der jeweils entsprechenden Formel (X), (XI) bzw. (XII) umsetzt, wenn für die Variante (A) als Ausgangsprodukt ein Racemat verwendet wird, die Verbindung der Formel (X) in die Diastereomeren trennt, und von den Verbindungen der Formel (X), (XI) bzw. (XII) die Acylgruppen $Ac^1$, $Ac^2$, $Ac^3$ und $Ac^4$ gleichzeitig abspaltet, wobei aus Verbindungen der D- bzw. der L-Reihe jeweils Verbindungen der D- bzw. der L-Reihe entstehen.

9. Verfahren nach Anspruch 8, dadurch gekennzeichnet, dass als Hydroxylschutzgruppe R eine räumlich grosse Gruppe wie die Triphenylmethyl-, Monomethoxytriphenylmethyl-, tert.Butyl-, Trichloracetyl-, Trimethylsilyl-, tert.Butyldimethylsilyl- oder tert.-Butyldiphenylsilylgruppe verwendet wird.

10. Verfahren nach Anspruch 8, dadurch gekennzeichnet, dass als Acylrest $Ac^1$ bzw. $Ac^3$ jener einer aliphatischen oder aromatischen Carbonsäure oder eine tert.Butoxycarbonylgruppe, vorzugsweise der Acylrest der Benzoesäure oder einer substituierten Benzoesäure, verwendet wird.

11. Verfahren nach Anspruch 8, dadurch gekennzeichnet, dass als Acylrest $Ac^2$ jener einer einfach zugänglichen optisch aktiven organischen Säure wie die Weinsäure, die Dibenzoylweinsäure, die Mandelsäure, die O-Acetylmandelsäure, die Camphersäure, die Camphersulfonsäure oder die Bromcamphersulfonsäure verwendet wird.

12. Verfahren nach Anspruch 8, dadurch gekennzeichnet, dass als Acylrest $Ac^4$ jener einer aliphatischen oder aromatischen Carbonsäure wie der Acetyl- oder Benzoylrest verwendet wird.

13. Verfahren nach Anspruch 8, dadurch gekennzeichnet, dass die Trennung des Gemisches diastereomerer Verbindungen der Formel (V) bzw. (X) in die Diastereomeren durch Chromatographie oder durch fraktionierte Kristallisation durchgeführt wird.

14. Verfahren nach Anspruch 8, dadurch gekennzeichnet, dass die Abspaltung des Acylrestes $Ac^2$ in der Variante (C) bzw. der Acylreste $Ac^1$, $Ac^2$, $Ac^3$ und $Ac^4$ in der letzten Verfahrensstufe durch Behandeln mit einem Alkalimetallalkoholat durchgeführt wird.

15. Verfahren nach Anspruch 8, dadurch gekennzeichnet, dass die Umsetzung mit dem Trifluor- oder Trichloracetimidat in Gegenwart einer Lewis-Säure wie Bortrifluorid-etherat oder Trifluormethansulfonsäuretrimethylsilylester in einem wasserfreien polaren Lösungsmittel durchgeführt wird.

16. Nach dem Verfahren gemäss Anspruch 8, hergestellte Sphingosinderivate der Formel (I)-D oder (I)-L.

E

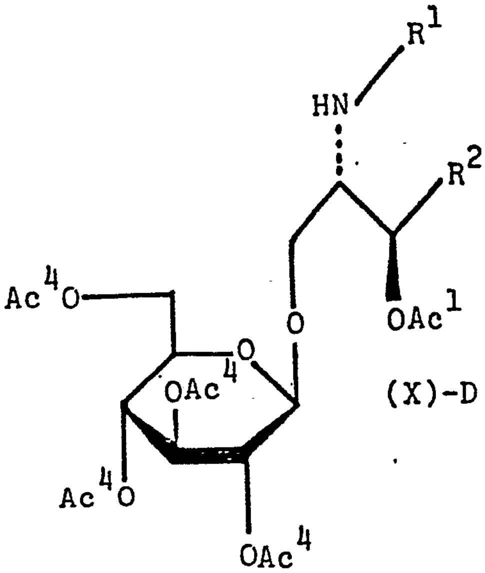

(X)-D

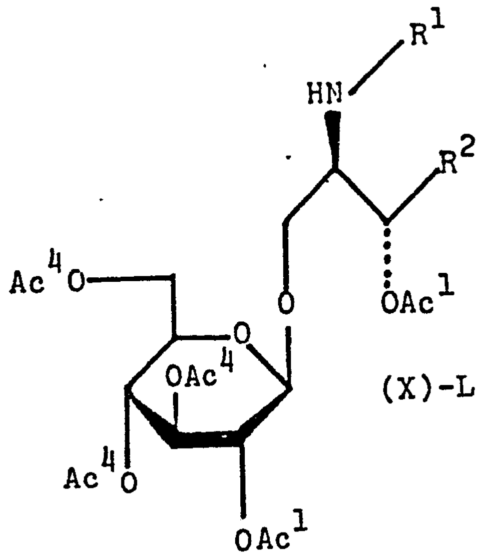

(X)-L

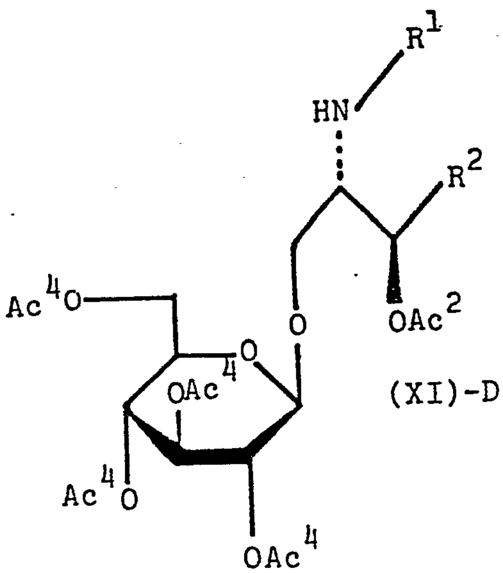

(XI)-D

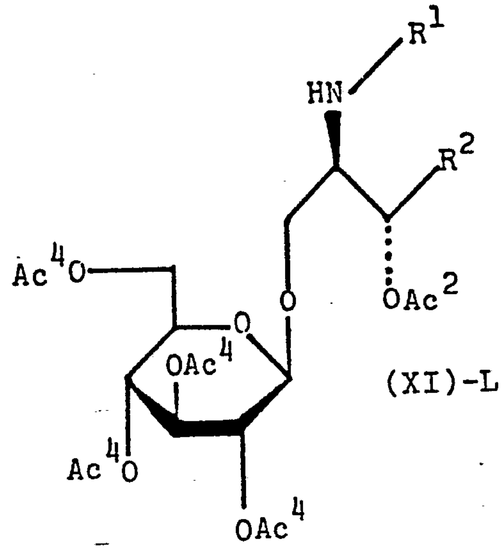

(XI)-L

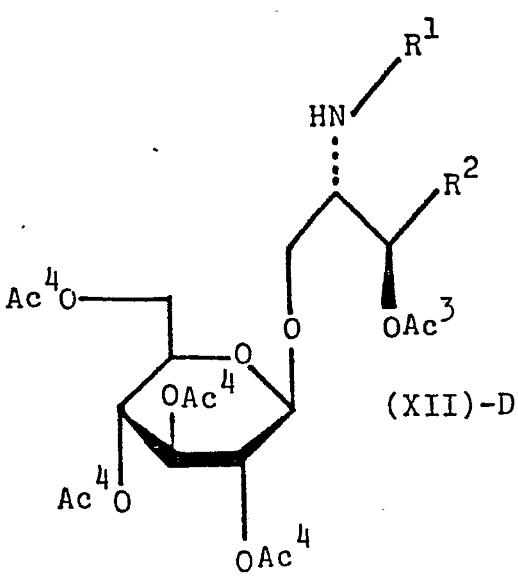

(XII)-D

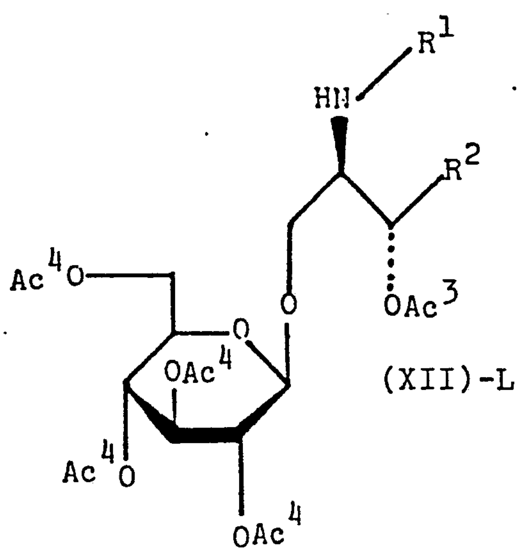

(XII)-L

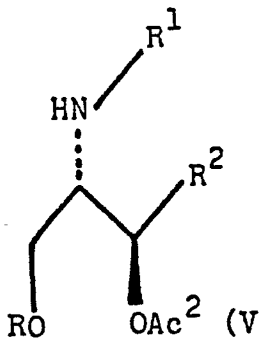
(V)-D

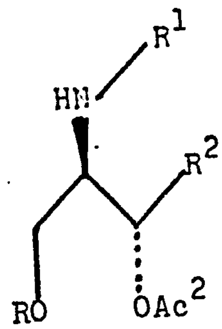
(V)-L

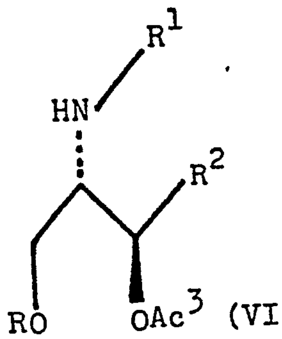
(VI)-D

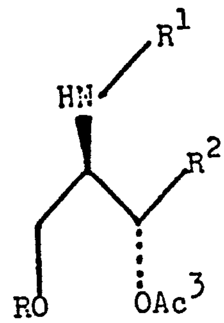
(VI)-L

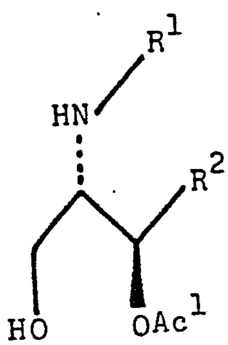
(VII)-D

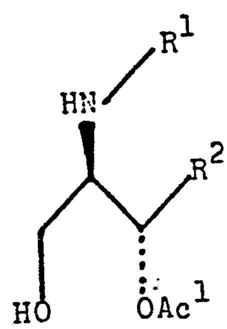
(VII)-L

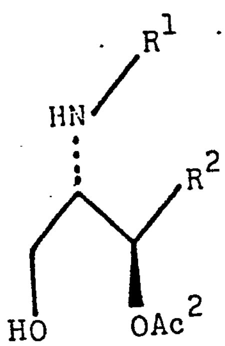
(VIII)-D

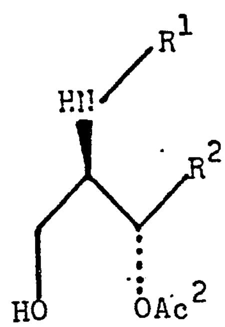
(VIII)-L

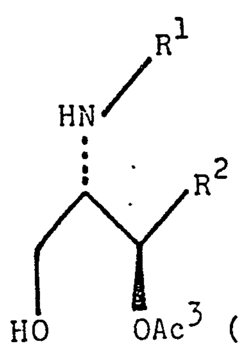
(IX)-D

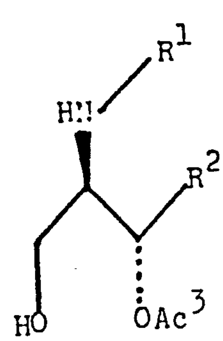
(IX)-L

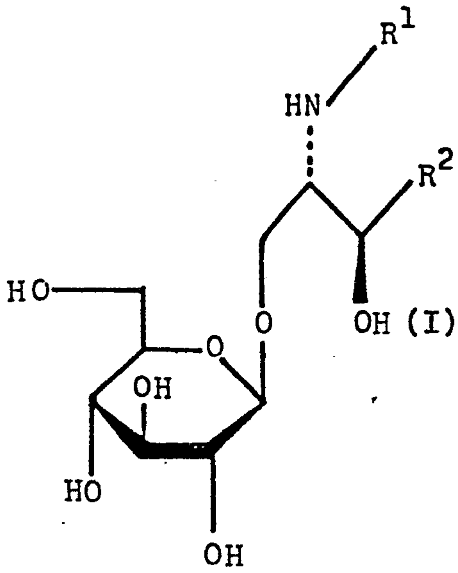

(I)-D

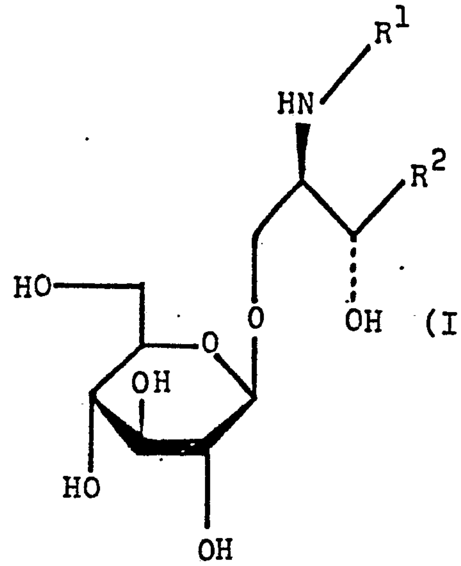

(I)-L

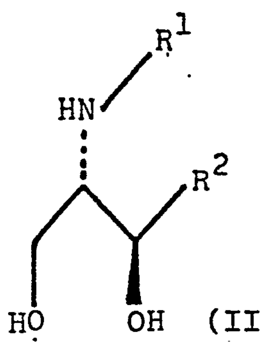

(II)-D

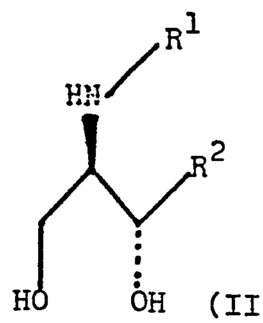

(II)-L

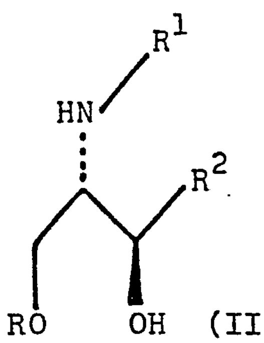

(III)-D

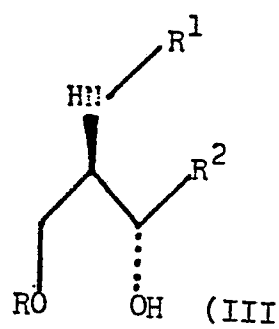

(III)-L

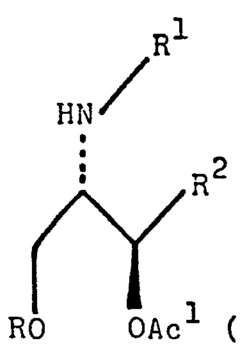

(IV)-D

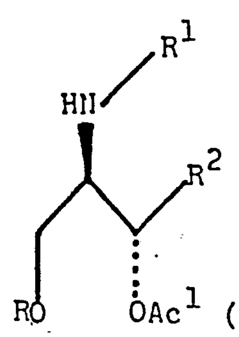

(IV)-L